# FASCICULE DE BREVET EUROPEEN

(11) **EP 3 154 615 B1**
(45) Date de publication et mention de la délivrance du brevet: **04.04.2018**
(21) Numéro de dépôt: 15732866.7
(22) Date de dépôt: 11.06.2015
(51) Int. Cl.: A61M 16/16, A61M 16/06, A61M 16/08, A61M 16/12, A61M 16/04, A61M 16/00

(54) **DISPOSITIF D'ASSISTANCE RESPIRATOIRE, APPAREIL NASAL ET MASQUE D'ASSISTANCE RESPIRATOIRE**
ATEMHILFEVORRICHTUNG, NASALE VORRICHTUNG UND ATEMHILFEMASKE
RESPIRATORY ASSISTANCE DEVICE, NASAL APPARATUS AND RESPIRATORY ASSISTANCE MASK

(30) Priorité: 13.06.2014 FR 1455395
(43) Date de publication de la demande: 19.04.2017
(73) Titulaire: Vygon, 95440 Ecouen (FR); Boussignac, Georges, 92160 Antony (FR)
(72) Inventeur: BOUSSIGNAC, Georges, 92160 Antony (FR); CARREZ, Jean-Luc, 95440 Ecouen (FR); BERTHEUIL, Elodie, 95430 Auvers sur Oise (FR); LESIMPLE BOBERT, Laurent, 93290 Tremblay en France (FR); RIGA, Cyril, 60600 Agnetz (FR)
(74) Mandataire: Jacobacci Coralis Harle
(86) Numéro de dépôt international: PCT/FR2015/051553
(87) Numéro de publication internationale: WO 2015/189525

(56) Documents cités:
- FR-A1- 2 813 197
- FR-A1- 2 973 708
- US-A1- 2014 053 841

## Description

La présente invention a pour objet un dispositif d'assistance respiratoire perfectionné, utilisable sur des patients dont la respiration spontanée est absente ou insuffisante, qu'ils soient placés ou non sous respiration artificielle. Elle est plus particulièrement adaptée aux dispositifs d'assistance respiratoire inventés par Monsieur Georges Boussignac.

Le principe à la base du dispositif d'assistance respiratoire inventé par Monsieur Boussignac consiste à injecter par un ensemble de canaux auxiliaires au moins un gaz respirable sous pression dans la lumière d'une tubulure reliée aux voies respiratoires du patient. Ce dispositif d'assistance a la particularité d'être du type ouvert, c'est-à-dire que la tubulure peut rester ouverte à l'air libre autorisant ainsi le passage de sondes ou autres accessoires de surveillance ou traitement du patient. Plus précisément, le dispositif d'assistance est formé d'un élément tubulaire ou tube qui forme un canal principal et qui est destiné à être relié par son extrémité distale à une voie respiratoire d'un patient pour que ledit canal principal relie, à l'extérieur, le système respiratoire dudit patient. Le dispositif comporte en outre au moins un canal auxiliaire permettant l'injection d'un jet de gaz respirable destiné à la ventilation dudit patient et débouchant par un orifice de sortie distal dans ledit canal principal au voisinage de l'extrémité distale de ce dernier. Dans ce dispositif d'assistance, en regard de l'orifice de sortie distal de chaque canal auxiliaire, sont prévus des moyens de déflexion desdits jets de gaz respirable(s) vers l'intérieur dudit canal principal.

Ainsi, le jet de gaz respirable sous pression traversant ledit canal auxiliaire est défléchi vers l'axe central du canal principal, lorsqu'il pénètre dans celui-ci. En général, plusieurs jets sont créés et défléchis les uns vers les autres vers l'axe central du canal principal. Les jets se rencontrent donc vers le centre du canal principal en y créant une zone de turbulence et de pression. En aval desdits moyens de déflexion, c'est-à-dire à l'intérieur du canal principal, la pression dudit jet de gaz respirable chute et le jet sort à faible pression à travers l'extrémité distale de l'élément tubulaire. L'expérience a montré qu'en aval de la sortie distale de l'élément tubulaire, la pression est faible et maintenue constante dans tout l'espace respiratoire. De plus, cette pression est dépendante du débit de gaz respirable dans les canaux auxiliaires et on a donc également un moyen simple de réglage de la pression en jouant sur le débit du gaz dans les canaux auxiliaires.

Ce dispositif d'assistance respiratoire peut ainsi permettre de maintenir une pression continue positive dans le système respiratoire du patient et évite un affaissement des parois des alvéoles pulmonaires préjudiciable à la ventilation. Une telle application de ce dispositif est dénommée CPAP pour « continuous positive airway pressure ».

Ce dispositif d'assistance respiration s'est également montré très utile dans le cadre de la réanimation cardiaque, plus généralement cardiopulmonaire, par massage thoracique externe manuel ou mécanique.

Ce dispositif d'assistance dont on pourra compléter la connaissance dans la demande de brevet FR89/04280 a fait l'objet de divers perfectionnements dans les demandes de brevets suivantes données à titre d'exemple non exhaustif : EP0390684, WO2008113913, EP2228088, FR2942967, WO2007118973, FR2911073, FR2813197, WO2003039638, FR2827778, EP0978291, FR2921840, FR2782925, FR2836384 et EP0701834.

On verra d'ailleurs que ces divers perfectionnements peuvent être mis en oeuvre dans le cadre de la présente invention. Notamment la nébulisation de produits liquides, l'aide à la réanimation, l'utilisation nasale, le double flux...

Ce dispositif a apporté un confort certain aux patients et au personnel soignant du fait qu'il est du type ouvert. De plus, il est relativement simple dans son principe et à fabriquer.

Toutefois, le dispositif tel qu'il a été proposé présente l'inconvénient de provoquer une nébulisation ou pulvérisation des expectorations ou des hémoptysies du patient lorsque celles-ci traversent la zone de turbulences où les jets défléchis se rencontrent dans le canal principal du dispositif de Monsieur Boussignac. Il existe donc un risque important de contamination du personnel si le patient est atteint d'une infection contagieuse. En particulier, lors de la réanimation cardiopulmonaire par massage thoracique externe manuel ou mécanique, des hémoptysies peuvent se produire. En outre, il peut arriver que des régurgitations se produisent et qui peuvent également être nébulisées ou pulvérisées.

Une solution simple serait l'ajout d'un filtre en sortie proximale du dispositif d'assistance respiratoire. Toutefois, cela implique de vérifier régulièrement sa perméabilité aux gaz, ce qui n'est pas toujours facile et évident dans des conditions de réanimation d'urgence.

On propose de réaliser une dérivation dans la zone des jets défléchis, à l'intérieur du canal principal, grâce à un tube intérieur creux de dérivation.

On a proposé dans l'état de la technique d'autres solutions présentées dans les documents FR 2 973 708, US 2014/053841 et FR 2 813 197 mais elles ne mettent pas en oeuvre des dérivations coaxiales pour les gaz expirés et/ou inspirés et limitées en étendue à la zone des jets défléchis.

L'invention concerne donc un dispositif d'assistance respiratoire selon la revendication 1.

Par rapport aux dispositifs connus qui peuvent comporter une tubulure interne pour aspiration de mucosités trachéopulmonaires ou gastriques et qui sont des sondes de grande longueur, le tube intérieur de l'invention est de longueur réduite, essentiellement limitée à la zone des jets défléchis de gaz et il n'est pas destiné à aller récupérer in situ des mucosités au cours d'aspirations ou un contenu de l'estomac ou à effectuer des mesures. Le tube intérieur est fixe et est compris dans le dispositif d'assistance respiratoire, essentiellement limité en longueur dans la zone des jets de gaz respirable(s) en sortie des orifices de sortie des canaux auxiliaires même s'il peut s'étendre un peu au-delà notamment pour des facilités de maintien/fixation. Le tube intérieur de l'invention est donc un tube brise jet et de dérivation.

Dans divers modes de mise en oeuvre de l'invention, les moyens suivants pouvant être utilisés seuls ou selon toutes les combinaisons techniquement possibles, sont employés :
- le dispositif d'assistance respiratoire est intégré ou rapporté sur au moins un élément de liaison, l'élément de liaison étant notamment un masque facial ou un adaptateur nasal, un masque laryngé, une tubulure de liaison distale à la voie respiratoires du patient, une tubulure de liaison distale d'étanchéité à ballonnet(s) gonflable(s), une tubulure de liaison proximale à un appareillage de ventilation,
- le dispositif d'assistance respiratoire est destiné à être mis en oeuvre à l'extérieur du patient, notamment intégré ou rapporté sur un masque facial ou sur un adaptateur nasal ou sur une tubulure de liaison distale à la voie respiratoires du patient,
- le dispositif d'assistance respiratoire est destiné à être mis en oeuvre à l'intérieur du patient, notamment intégré ou rapporté sur un masque laryngé ou sur une tubulure de liaison distale à la voie respiratoires du patient ou sur une tubulure de liaison distale d'étanchéité à ballonnet(s) gonflable(s) dont intratrachéale,
- le canal principal est cylindrique,
- le/les canaux auxiliaires sont réalisés dans l'épaisseur de la paroi de l'élément tubulaire,
- le dispositif comporte un ensemble de canaux auxiliaires, lesdits canaux auxiliaires étant sensiblement parallèles à l'axe central dudit élément tubulaire sur au moins une partie de leurs trajets,
- le/les orifices de sortie distaux du/des canaux auxiliaires débouchent au niveau de la surface interne de l'élément tubulaire,
- l'élément tubulaire comporte au sein de sa paroi, en outre du/des canaux auxiliaires permettant l'injection de jet(s) de gaz respirable(s), au moins un canal auxiliaire annexe permettant l'injection dans le canal principal par un orifice de sortie distal annexe du canal auxiliaire annexe de produits distincts du gaz respirable(s), notamment de liquides,
- l'élément tubulaire comporte au sein de sa paroi, en outre du/des canaux auxiliaires permettant l'injection de jet(s) de gaz respirable(s) et en outre du/des éventuels canaux auxiliaires annexes, au moins un conduit d'apport permettant le passage vers le patient de fluides, notamment gazeux ou liquides,
- l'élément tubulaire comporte au sein de sa paroi, en outre du/des canaux auxiliaires permettant l'injection de jet(s) de gaz respirable(s) et en outre du/des éventuels canaux auxiliaires annexes, au moins un conduit de mesure permettant de récupérer du patient des échantillons de fluides, notamment gaz expirés ou sécrétions, vers un appareillage de prélèvement et/ou d'aspiration et/ou de mesure externe,
- l'élément tubulaire comporte au sein de sa paroi, en outre du/des canaux auxiliaires permettant l'injection de jet(s) de gaz respirable(s) et en outre du/des éventuels canaux auxiliaires annexes, au moins un conduit de mesure permettant de mesurer la pression des gaz respiratoires côté distal du dispositif,
- le tube intérieur est cylindrique,
- la surface externe du tube intérieur est sensiblement lisse,
- la surface externe du tube intérieur comporte des éléments en relief et en creux, notamment dans la zone où les axes des jets défléchis rencontrent ledit tube intérieur,
- le tube intérieur est maintenu en position coaxiale à l'intérieur du canal principal par des ailettes étendues radialement entre la surface externe du tube intérieur et la surface interne de l'élément tubulaire, lesdites ailettes étant absentes dans la zone des jets défléchis,
- les ailettes sont d'épaisseur réduite,
- un corps tubulaire est rapporté à l'intérieur de l'élément tubulaire,
- de préférence, le corps tubulaire est rapporté côté proximal de l'élément tubulaire,
- un corps tubulaire est rapporté côté distal de l'élément tubulaire,
- le tube intérieur est maintenu en position coaxiale à l'intérieur du canal principal par un seul ensemble d'ailettes étendues radialement entre la surface externe du tube intérieur et la surface interne de l'élément tubulaire ou d'un corps tubulaire rapporté proximal, ledit ensemble d'ailettes étant du côté de l'extrémité proximale de l'élément tubulaire, lesdites ailettes étant absentes dans la zone des jets défléchis,
- le tube intérieur est maintenu en position coaxiale à l'intérieur du canal principal par un seul ensemble d'ailettes étendues radialement entre la surface externe du tube intérieur et la surface interne de l'élément tubulaire ou d'un corps tubulaire rapporté distal, ledit ensemble d'ailettes étant du côté de l'extrémité distale de l'élément tubulaire, lesdites ailettes étant absentes dans la zone des jets défléchis,
- le tube intérieur est maintenu en position coaxiale à l'intérieur du canal principal par deux ensembles d'ailettes étendues radialement entre la surface externe du tube intérieur et la surface interne de l'élément tubulaire et/ou d'un corps tubulaire rapporté, un premier ensemble d'ailettes étant du côté de l'extrémité proximale de l'élément tubulaire et le second ensemble d'ailettes étant du côté de l'extrémité distale de l'élément tubulaire, lesdites ailettes étant absentes dans la zone des jets défléchis,
- les ailettes et le tube intérieur forment une pièce monobloc insérée dans l'élément tubulaire ou dans un corps tubulaire lui-même inséré dans l'élément tubulaire,
- l'extrémité proximale du tube intérieur est au niveau de la/des orifices de sortie distaux du/des canaux auxiliaires,
- l'extrémité proximale du tube intérieur est au niveau de l'extrémité proximale dudit dispositif d'assistance respiratoire,
- l'extrémité proximale du tube intérieur est en retrait de l'extrémité proximale dudit dispositif d'assistance respiratoire, à l'intérieur du canal principal,
- les moyens de déflexion sont configurés de manière à permettre d'orienter les jets vers l'extrémité distale de l'élément tubulaire selon une incidence par rapport à l'axe central dudit élément tubulaire, et donc par rapport au tube intérieur coaxial, comprise entre 90°, l'axe des jets étant perpendiculaire audit axe central, et 25°, l'axe des jets croisant l'axe central selon un angle de 25°,
- l'inclinaison des jets défléchis est différente de 90° en référence à l'axe central dudit élément tubulaire, les jets défléchis n'étant pas radiaux mais inclinés vers l'extrémité distale et le centre du canal principal, et l'extrémité proximale du tube intérieur est au niveau de la/des orifices de sortie distaux du/des canaux auxiliaires,
- les moyens de déflexion permettent de faire converger les uns vers les autres, à l'intérieur du canal principal, les jets de gaz respirable(s) des canaux auxiliaires,
- tous les jets ont la même incidence,
- les jets ont des incidences différentes selon les orifices de sortie,
- l'incidence des jets est d'environ 45°,
- la pièce monobloc à ailettes et tube intérieur est introduite dans l'élément tubulaire par l'extrémité proximale de ce dernier, les ailettes étant disposées seulement du côté de l'extrémité proximale dudit élément tubulaire et formant en outre un obstacle à un bouchage du canal principal,
- l'obstacle au bouchage du canal principal n'empêche pas le passage éventuel d'une sonde, par exemple sonde d'aspiration, de mesure ou de prélèvement,
- la pièce monobloc à ailettes et tube intérieur comporte à la périphérie radiale des ailettes, notamment pour chaque ensemble d'ailettes, un anneau circulaire dont la surface extérieure est au contact de la surface interne de l'élément tubulaire ou d'un corps tubulaire rapporté,
- la pièce monobloc à ailettes et tube intérieur comporte à la périphérie radiale des ailettes, notamment pour chaque ensemble d'ailettes, un anneau circulaire dont la surface extérieure est au contact de la surface interne d'un corps tubulaire inséré dans l'élément tubulaire,
- l'élément tubulaire comporte des éléments structuraux destinés à former à eux seuls ou en combinaison avec d'autres éléments structuraux de l'anneau circulaire ou du corps tubulaire, en partie ou totalité, au moins le/les canaux auxiliaires,
- le corps tubulaire comporte des éléments structuraux destinés à former à eux seuls ou en combinaison avec d'autres éléments structuraux de l'élément tubulaire, en partie ou totalité, au moins le/les canaux auxiliaires,
- l'anneau circulaire de la pièce monobloc ou le corps tubulaire comporte des éléments structuraux destinés à former à eux seuls ou en combinaison avec d'autres éléments structuraux de l'élément tubulaire, en partie ou totalité, le/les canaux auxiliaires et une partie du/des orifices de sortie des canaux auxiliaires,
- l'anneau circulaire est d'épaisseur réduite,
- dans le cas d'une pièce monobloc à ailettes, tube intérieur et anneau circulaire disposée à l'extrémité proximale de l'élément tubulaire, ledit l'anneau circulaire est de longueur telle que son extrémité distale se termine avant ou juste au niveau du/des orifices de sortie distaux du/des canaux auxiliaires,
- l'anneau circulaire, dans le cas d'une pièce monobloc disposée côté de l'extrémité proximale de l'élément tubulaire, comporte des éléments structuraux destinés à former à eux seuls ou en combinaison avec d'autres éléments structuraux de l'élément tubulaire, en partie ou totalité, au moins le/les canaux auxiliaires,
- la pièce monobloc à ailettes et tube intérieur comporte à la périphérie radiale des ailettes un anneau circulaire dont la surface extérieure est au contact de la surface interne de l'élément tubulaire, ledit l'anneau circulaire étant de longueur telle que son extrémité distale se termine au niveau du/des orifices de sortie distaux du/des canaux auxiliaires, et l'anneau circulaire comporte des éléments structuraux destinés à former à eux seuls ou en combinaison avec d'autres éléments structuraux de l'élément tubulaire, en partie ou totalité, au moins le/les canaux auxiliaires,
- le/les orifices de sortie distaux du/des canaux auxiliaires sont ponctuels individuels,
- le diamètre de chaque orifice de sortie ponctuel est inférieur ou égal à 150 microns,
- le diamètre de chaque orifice de sortie ponctuel est inférieur ou égal à 100 microns,
- le diamètre de chaque orifice de sortie ponctuel est compris entre 50 microns et 10 microns et est, de préférence d'environ 25 microns,
- le diamètre de chaque orifice de sortie ponctuel est compris entre 20 microns et 5 microns et est, de préférence d'environ 10 microns,
- le diamètre de chaque orifice de sortie ponctuel est compris entre 150 microns et 10 microns,
- le diamètre de chaque orifice de sortie ponctuel est compris entre 150 microns et 100 microns,
- les canaux auxiliaires ont sur au moins une partie de leurs trajets un diamètre sensiblement égal à celui de leurs orifices de sortie,
- un canal auxiliaire donné débouche sur un seul orifice de sortie,
- un canal auxiliaire donné débouche sur plusieurs orifices de sortie,
- les orifices de sortie sont aux extrémités distales des canaux auxiliaires,
- les orifices de sortie sont décalés par rapport aux extrémités distales des canaux auxiliaires, lesdites extrémités distales des canaux auxiliaires au-delà des orifices de sortie étant en cul de sac,
- les diamètres de l'orifice de sortie et du canal auxiliaire correspondant sont identiques,
- au moins la/les parties distales du/des canaux auxiliaires débouchant dans le canal principal est/sont parallèles à celui-ci,
- l'orifice de sortie du canal auxiliaire est dans un évidement de la paroi interne de l'élément tubulaire, ledit évidement formant moyen de déflexion,
- le/les canaux auxiliaires sont ménagés dans la paroi de l'élément tubulaire,
- le dispositif comporte plusieurs canaux auxiliaires, chaque canal auxiliaire formant un passage tubulaire dans la paroi de l'élément tubulaire,
- le dispositif comporte un seul canal auxiliaire commun sur lequel le/les orifices de sortie sont reliés, ledit canal auxiliaire commun étant un passage annulaire ou une bague de distribution, externe et sensiblement coaxial et parallèle à l'axe central dudit élément tubulaire,
- au moins certains des orifices de sortie sont réunis ensembles en formant un orifice de sortie unique en bande de forme annulaire ou en segment d'anneau et dont la largeur/épaisseur d'ouverture/orifice correspond au diamètre d'un orifice ponctuel individuel, soit une largeur/épaisseur inférieure à 150 microns,
- dans le cas d'un seul canal auxiliaire formant un passage annulaire coaxial au canal principal, les orifices de sortie sont réunis ensembles en formant une sortie annulaire unique débouchant dans le canal principal, la sortie annulaire ayant une largeur/épaisseur correspondant au diamètre d'un orifice ponctuel individuel,
- les orifices de sortie sont choisis parmi des orifices ponctuels individualisés et/ou des orifices de sortie réunis en une ouverture/orifice en bande de préférence annulaire ou semi-annulaire,
- le dispositif comporte un orifice de sortie de canaux auxiliaires en bande annulaire,
- le dispositif comporte plusieurs orifices de sortie de canaux auxiliaires en bandes semi-annulaires,
- le/les orifices de sortie distaux du/des canaux auxiliaires sont en bandes semi-annulaires d'épaisseur réduite,
- le/les orifices de sortie distaux du/des canaux auxiliaires sont pratiquement un orifice en bande annulaire d'épaisseur réduite le long de la circonférence interne de l'élément tubulaire,
- l'épaisseur de chaque orifice de sortie en bande annulaire ou semi-annulaire est inférieure à 150 microns,
- l'épaisseur de chaque orifice de sortie en bande annulaire ou semi-annulaire est comprise entre 50 microns et 10 microns et est, de préférence d'environ 25 microns,
- le dispositif comporte en outre du/des orifices de sortie en bandes semi-annulaires des orifices de sortie ponctuels individualisés,
- le dispositif comporte en outre du/des orifices de sortie en bandes semi-annulaires des orifices de sortie ponctuels, lesdits orifices ponctuels ayant un diamètre inférieur à 150 microns,
- le/les orifices de sortie en bande(s) résultent de la réunion d'au moins certains des orifices de sortie distaux et de leurs canaux auxiliaires correspondants, les canaux auxiliaires concernés étant donc en forme de bande annulaire ou semi-annulaire,
- dans le cas où le/les orifices de sortie sont en bande(s) annulaire ou semi-annulaire, l'anneau circulaire de la pièce monobloc comportant des éléments structuraux destinés à former à eux seuls ou en combinaison avec d'autres éléments structuraux de l'élément tubulaire, en partie ou totalité, au moins le/les canaux auxiliaires, forme en outre une partie du/des orifices de sortie des canaux auxiliaires,
- l'anneau circulaire comportant des éléments structuraux destinés à former à eux seuls ou en combinaison avec d'autres éléments structuraux de l'élément tubulaire, en partie ou totalité, au moins le/les canaux auxiliaires, forme en outre une partie du/des orifices de sortie des canaux auxiliaires,
- dans le cas où le/les orifices de sortie sont en bande(s) annulaire ou semi-annulaire, le corps tubulaire comportant des éléments structuraux destinés à former à eux seuls ou en combinaison avec d'autres éléments structuraux de l'élément tubulaire, en partie ou totalité, au moins le/les canaux auxiliaires, forme en outre une partie du/des orifices de sortie des canaux auxiliaires,
- le corps tubulaire comportant des éléments structuraux destinés à former à eux seuls ou en combinaison avec d'autres éléments structuraux de l'élément tubulaire, en partie ou totalité, au moins le/les canaux auxiliaires, forme en outre une partie du/des orifices de sortie des canaux auxiliaires,
- le/les orifices de sortie distaux du/des canaux auxiliaires sont ponctuels et/ou sont en bande(s) annulaire ou semi-annulaires, et le diamètre des orifices ponctuels ou l'épaisseur des orifices en bande(s) est inférieure à 150 microns.
- dans le cas d'orifices de sortie réunis, les canaux auxiliaires correspondant sont également réunis en formant un canal auxiliaire annulaire ou semi-annulaire sur la totalité de leurs longueurs ou une partie seulement,
- l'orifice de sortie distal de jet est formé dans une première face s'écartant dudit canal principal et les moyens de déflexion sont formés par une seconde face, inclinée, dudit canal principal, disposée en regard de ladite première face et convergente en direction de l'orifice de sortie,
- la première face est sur l'élément tubulaire,
- la seconde face est sur l'élément tubulaire ou sur le corps tubulaire,
- la portion terminale du canal principal côté extrémité distale est évasée,
- la seconde face est prolongée vers l'extrémité distale du canal principal par une paroi évasant légèrement ledit canal principal,
- les moyens de déflexion sont formés directement dans la paroi interne de l'élément tubulaire,
- les moyens de déflexion sont formés sur un embout rapporté à l'extrémité distale de l'élément tubulaire,
- les moyens de déflexion consistent en un ensemble discontinu d'évidements de forme générale conique, ménagés dans la paroi interne, et au fond de chacun desquels débouche l'extrémité distale d'un canal auxiliaire par son orifice de sortie,
- le dispositif comporte une pluralité de canaux auxiliaires dont au moins certains sont alimentés en commun en gaz respirable sous pression,
- les canaux auxiliaires non alimentés en commun, servent à l'introduction de produits gazeux additionnels, tels que des produits médicamenteux ou des gaz humides,
- au moins une partie des canaux auxiliaires sont alimentés en commun en gaz respirable par l'intermédiaire d'une bague de distribution, coaxiale à l'élément tubulaire,
- au moins une autre partie des canaux auxiliaires sont alimentés en commun par des produits médicamenteux ou de l'humidité,
- la portion terminale du canal principal côté extrémité distale comporte une partie rétrécie,
- la portion terminale du canal principal côté extrémité distale comporte une bague de rétrécissement,
- l'extrémité distale du tube intérieur est au niveau de l'extrémité distale dudit dispositif d'assistance respiratoire,
- l'extrémité distale du tube intérieur est en retrait de l'extrémité distale dudit dispositif d'assistance respiratoire, à l'intérieur du canal principal,
- l'extrémité distale du tube intérieur est réalisée à une distance déterminée de la zone d'arrivée sur le tube intérieur des axes des jets défléchis sortant directement des orifices, ladite distance déterminée étant d'au moins 5 mm,
- l'extrémité distale du tube intérieur est réalisée à une distance déterminée de la zone d'arrivée sur le tube intérieur des axes des jets défléchis sortant directement des orifices, ladite distance déterminée étant de préférence d'au moins 10 mm,
- l'extrémité distale du tube intérieur est réalisée à une distance déterminée de la zone d'arrivée sur le tube intérieur des axes des jets défléchis sortant directement des orifices, ladite distance déterminée étant plus préférentiellement d'au moins 15 mm,
- l'extrémité distale du tube intérieur est réalisée à une distance déterminée de la zone d'arrivée sur le tube intérieur des axes des jets défléchis sortant directement des orifices, ladite distance déterminée étant plus particulièrement de 16 mm,
- le tube intérieur a une longueur d'environ 33,5 mm,
- le tube intérieur a plus particulièrement une longueur d'environ 43,5 mm,
- les ailettes ont des longueurs inférieures à la longueur du tube intérieur,
- l'extrémité distale du tube intérieur est approximativement au niveau de l'extrémité distale dudit dispositif d'assistance respiratoire et l'extrémité proximale du tube intérieur est approximativement au niveau de l'extrémité proximale dudit dispositif d'assistance respiratoire,
- dans le dispositif d'assistance respiratoire, la distance transversale entre les orifices de sortie de canaux auxiliaires et la surface externe du tube intérieur est comprise entre 1 mm et 5 mm,
- les orifices de sortie de canaux auxiliaires sont disposés en couronne,
- le dispositif comporte au moins une couronne d'orifices de sortie de canaux auxiliaires, les orifices de sortie d'une couronne étant disposés le long d'une section transversale dudit élément tubulaire,
- le dispositif comporte au moins deux couronnes d'orifices de sortie de canaux auxiliaires décalés le long dudit élément tubulaire, les orifices de sortie de couronnes différentes provenant de canaux auxiliaires identiques, un même canal auxiliaire pouvant déboucher sur plusieurs orifices de sortie,
- le dispositif comporte au moins deux couronnes d'orifices de sortie de canaux auxiliaires décalés le long dudit élément tubulaire, les orifices de sortie de couronnes différentes provenant de canaux auxiliaires différents, un canal auxiliaire donné ne débouchant que sur un seul orifice de sortie,
- les orifices de sortie d'une couronne ou une partie de ceux-ci sont réunis pour former un orifice commun annulaire ou semi annulaire,
- le dispositif comporte au moins deux couronnes d'orifices de sortie en bandes annulaires ou semi annulaires,
- le dispositif à couronnes d'orifices comporte en outre de son/ses orifices de sortie en bandes annulaires et/ou semi-annulaires des orifices de sortie ponctuels,
- les jets ont des incidences différentes selon la couronne d'orifices de sortie,
- le dispositif comporte un manomètre relié au canal principal pour mesure de pression,
- le point de mesure de pression du manomètre relié au canal principal est situé vers l'extrémité distale du dispositif,
- le point de mesure de pression du manomètre relié au canal principal est situé vers l'extrémité proximale du dispositif,
- le dispositif comporte un manomètre relié au conduit intérieur du tube brise jet creux de dérivation,
- le dispositif comporte intérieurement un indicateur visuel de direction et force du flux gazeux passant par le dispositif,
- ledit indicateur est dans le canal principal du dispositif,
- ledit indicateur est situé vers l'extrémité distale du dispositif,
- ledit indicateur est situé vers l'extrémité proximale du dispositif,
- ledit indicateur est un élément s'orientant dans le sens du flux gazeux,
- ledit indicateur est un élément souple s'orientant dans le sens du flux gazeux et se déformant en fonction de la force du flux gazeux,
- ledit indicateur est une bandelette souple en matière plastique fixée à une de ses deux extrémités à une ailette de maintien du tube intérieur,
- lorsque le flux gazeux est expiratoire, la bandelette est orientée vers l'extrémité proximale du dispositif et, en fonction de sa longueur, peut en sortir par ladite extrémité proximale,
et/ou, dans certaines variantes :
- le dispositif est à double flux inversés permettant de favoriser l'expiration aussi bien que l'inspiration d'un patient, le dispositif comportant au moins un canal auxiliaire supplémentaire, indépendant du/des premiers canaux auxiliaires des jets de l'extrémité distale du canal principal, et relié à une source de gaz sous pression, ledit au moins un canal auxiliaire supplémentaire débouchant dans le canal principal au voisinage de l'extrémité proximale de ce dernier, tandis que, en regard de chaque orifice de sortie du canal auxiliaire supplémentaire correspondant, sont prévus des moyens pour la déflexion du jet de gaz traversant ce dernier en direction de l'intérieur dudit canal principal afin de former des jets d'extrémité proximale du canal principal, et les deux zones de jets défléchis correspondant respectivement aux jets de l'extrémité distale du canal principal et aux jets de l'extrémité proximale du canal principal comportent chacune un tube intérieur brise jet et de dérivation, ledit tube intérieur pouvant être étendu pour être unique et commun aux deux zones ou chacune des zones comportant son tube intérieur propre,
- au moins l'/les extrémités dudit/desdits canaux auxiliaires supplémentaires débouchant par des orifices de sortie respectifs dans le canal principal est/sont parallèle à celui-ci,
- de préférence, dans le cas d'un dispositif double flux avec canal auxiliaire supplémentaire, la partie proximale du dispositif est semblable sinon identique, symétriquement, à la partie distale dudit dispositif, au moins en ce qui concerne la disposition desdits canaux auxiliaires et lesdits moyens de déflexion,
- le dispositif constitue l'embout d'entrée et de sortie d'air d'un masque d'assistance respiratoire destiné à être appliqué sur le visage d'un patient,
- le dispositif constituant l'embout d'entrée et de sortie d'air d'un masque d'assistance respiratoire est amovible,
et/ou, dans certaines variantes :
- le dispositif comporte en outre des moyens de dérivation aptes à dériver une fraction de volume dudit gaz respirable destiné audit canal auxiliaire avant son entrée dans ce dernier et des moyens d'aspiration d'air ambiant entraînés par ladite fraction dérivée de gaz respirable et lesdits moyens d'aspiration sont reliés au canal principal de sorte que les moyens d'aspiration sont aptes à acheminer, dans ledit canal principal l'air ambiant aspiré mélangé à ladite fraction dérivée de gaz respirable,
- le dispositif comporte des moyens de réglage de la fraction de gaz respirable dérivée par lesdits moyens de dérivation,
- les moyens de réglage de la fraction de gaz respirable dérivée sont disposés entre lesdits moyens de dérivation et les moyens d'aspiration d'air ambiant,
- les moyens de réglage de la fraction de gaz respirable dérivée comportent au moins une vanne,
- le dispositif comporte en outre des moyens de régulation de débit de gaz respirable dilué sortant desdits moyens d'aspiration et destiné à entrer dans ledit canal principal,
- les moyens de régulation de débit de gaz respirable dilué sont agencés entre lesdits moyens d'aspiration d'air ambiant et des moyens de communication fluidique,
- les moyens de régulation de débit de gaz respirable dilué comportent au moins une vanne,
- le débit de gaz respirable dilué sortant desdits moyens d'aspiration passe dans le canal principal par un orifice de communication qui est ménagé dans la paroi dudit dispositif,
- le canal principal est formé par un tube,
- le canal principal est formé par un tube souple,
- le débit de gaz respirable dilué sortant desdits moyens d'aspiration passe par une gaine souple étanche qui enveloppe, au moins sur une partie de sa longueur ledit tube souple formant le canal principal et qui forme une voie périphérique autour dudit tube souple et dans laquelle débouche l'orifice de communication,
- les moyens d'aspiration sont montés directement sur ledit tube souple au voisinage de son extrémité proximale,
- l'orifice de communication est disposé entre les moyens de déflexion des jets et l'extrémité distale du canal principal,
- l'orifice de communication est divisé en des orifices de communication multiples,
et/ou, dans certaines variantes :
- le dispositif comporte en outre des moyens de freinage de sortie des gaz d'expiration du patient,
- le dispositif comporte en outre des moyens de freinage d'entrée de gaz d'inspiration de l'extérieur vers le patient,
- les moyens de freinage sont disposés côté proximal du dispositif,
- les moyens de freinage sont intégrés au dispositif,
- les moyens de freinage sont rapportés par connexion sur l'extrémité proximale du dispositif,
- les moyens de freinage comportent des soupapes,
- les moyens de freinage sont configurés pour ne freiner le passage des gaz que pendant au moins une phase déterminée d'une inspiration et/ou d'une expiration et de préférence pendant une phase d'inspiration qui est le début de l'inspiration afin de provoquer une dépression intrathoracique favorable au retour veineux vers le coeur,
- le dispositif comporte en outre des moyens de freinage d'entrée de gaz d'inspiration de l'extérieur vers le patient et les moyens de freinage sont configurés pour ne freiner le passage des gaz que pendant une phase d'inspiration qui est le début de l'inspiration afin de provoquer une dépression intrathoracique favorable au retour veineux vers le coeur,
- le dispositif comporte en outre des moyens de freinage spontané de l'entrée d'air extérieur dans le canal principal via son extrémité proximale,
- les moyens de freinage de l'entrée d'air extérieur dans le canal principal comportent un corps creux pourvu d'une première et d'une seconde soupapes normalement fermées, la première soupape étant apte à s'ouvrir spontanément et immédiatement lors d'une surpression/contrepression provenant de la voie respiratoire du patient, notamment lors d'une compression thoracique, alors que la seconde soupape est apte à s'ouvrir spontanément, mais progressivement, en dehors de ladite surpression/contrepression, notamment lors de la suppression d'une compression thoracique, et le corps creux est disposé à l'extrémité proximale de l'élément tubulaire du dispositif,
- les première et seconde soupapes sont disposées en parallèle entre l'extérieur et la cavité interne du corps creux,
- les première et seconde soupapes sont disposées en série entre l'extérieur et la cavité interne du corps creux, l'une desdites soupapes étant portée par l'autre,
- la première soupape est constituée par une membrane élastique s'appliquant spontanément contre un siège prévu dans ledit corps creux et est liée audit siège par des points de fixation répartis à sa périphérie, l'air chassé lors des surpression/contrepression passant librement de la cavité du corps creux à l'extérieur par des passages qui se forment spontanément et immédiatement par la déformation élastique de ladite membrane entre lesdits points de fixation et ledit siège et la seconde soupape est formée par au moins une fente à bords jointifs pratiquée dans ladite membrane, l'air aspiré lors de la suppression de la surpression/contrepression passant progressivement en étant freiné de l'extérieur à la cavité du corps creux par le passage qui se forme spontanément dans ladite membrane par déformation élastique de celle-ci entraînant l'écartement progressif de ses bords jointifs,
- les moyens de freinage de l'entrée d'air extérieur dans l'élément tubulaire du dispositif font partie intégrante de celui-ci,
- les moyens de freinage de l'entrée d'air extérieur dans l'élément tubulaire du dispositif sont rapportés de façon amovible à celui-ci,
et/ou, dans certaines variantes :
- l'élément tubulaire formant le canal principal du dispositif comporte au moins un orifice latéral de sécurité qui traverse sa paroi latérale au moins sensiblement en regard du point de convergence des axes des jets de gaz respirable(s) et qui est apte à relier à l'extérieur la partie dudit canal principal qui se trouve côté distal/en aval par rapport au sens des jets de gaz respirable(s) et par rapport aux moyens de déflexion,
- la partie distale du canal principal est en communication directe avec l'extérieur à travers ledit orifice latéral de sécurité,
- l'orifice latéral de sécurité est fermé par un bouchon amovible,
- l'orifice latéral de sécurité est fermé par un bouchon amovible imperdable,
- l'orifice latéral de sécurité est fermé par une soupape d'échappement tarée s'ouvrant en cas de surpression,
- le dispositif comporte des moyens fibreux ou poreux pour masquer le bruit des jets de gaz respiratoire traversant ledit orifice latéral de sécurité,
- le dispositif comporte un conduit reliant l'orifice latéral de sécurité à l'extérieur,
- les moyens fibreux ou poreux sont dans le conduit,
- le conduit est constitué par un conduit coaxial entourant l'élément tubulaire,
- le conduit coaxial débouche à l'extérieur du côté de l'extrémité proximale dudit élément tubulaire,
- le conduit coaxial débouche à l'extérieur du côté de l'extrémité distale dudit élément tubulaire,
- le conduit est constitué par une gaine souple entourant l'élément tubulaire et débouchant à l'extérieur du côté de l'extrémité proximale dudit élément tubulaire,
- le conduit est constitué par une gaine souple entourant l'élément tubulaire et débouchant à l'extérieur du côté de l'extrémité distale dudit élément tubulaire,
- la partie proximale du canal principal comporte des obstacles internes saillants empêchant l'obturation hermétique de l'extrémité proximale,
et/ou, dans certaines variantes :
- afin que le dispositif puisse délivrer à la voie respiratoire du patient un débit prédéterminé de gaz respiratoire(s) sous une pression d'utilisation dont la valeur doit être comprise dans une plage de valeurs d'utilisation pour assurer l'efficacité dudit dispositif sans mettre en danger ledit patient, ledit dispositif étant relié à la source de gaz respiratoire(s) par un conduit d'alimentation de gaz et à une pression d'alimentation comprise entre une valeur minimale et une valeur maximale, le conduit d'alimentation en gaz respiratoire(s) comporte un élément de perte de charge spécifique assurant que ladite pression d'utilisation est au plus égale à la valeur supérieure de ladite plage de valeurs d'utilisation, lorsque ladite pression d'alimentation est à ladite valeur maximale,
- l'élément de perte de charge spécifique est constitué par un bouchon apte à obturer le conduit d'alimentation en gaz respiratoire et percé d'un passage longitudinal pour le/les gaz,
- la valeur de la perte de charge apportée par ledit élément de perte de charge spécifique est ajustée par la longueur du bouchon percé,
- l'élément de perte de charge spécifique est constitué par un tronçon d'un profilé,
et/ou, dans certaines variantes :
- le dispositif comporte entre les moyens de déflexion et l'extrémité distale du canal principal, des moyens de communication commandables à l'ouverture et à la fermeture et aptes, lorsqu'ils sont en position ouverte, à former un passage reliant ledit canal principal à l'environnement extérieur,
- le passage reliant le canal principal à l'environnement extérieur a une section variable,
- les moyens de communication sont du type à bague tournante percée latéralement et apte à découvrir des passages de diamètres différents,
- la bague tournante est montée directement sur l'élément tubulaire,
- la bague tournante est montée sur une cheminée en communication avec le canal principal,
et/ou, dans certaines variantes :
- le dispositif comporte en outre au moins un conduit d'apport alimenté en produit liquide à nébuliser dans le canal principal et des moyens de nébulisation,
- le dispositif comporte en outre au moins un conduit d'apport rapporté alimenté en produit liquide à nébuliser et logé dans le canal principal, ledit conduit d'apport pénétrant dans le canal principal à travers l'extrémité proximale de l'élément tubulaire et débouchant dans ledit canal principal au voisinage du débouché, dans ce dernier, du/des orifices de sortie du/des canaux auxiliaires,
- le diamètre interne dudit conduit d'apport rapporté est de l'ordre de 200 à 300 microns,
- à l'extérieur de l'élément tubulaire, le conduit d'apport rapporté est ascendant et au moins approximativement perpendiculaire à l'axe de l'élément tubulaire,
- le conduit d'apport est rapporté à l'élément tubulaire de façon amovible,
- le conduit d'apport est en outre fixé à l'élément tubulaire au moyen d'une pince disposée à cheval sur le bord de l'extrémité proximale de l'élément tubulaire,
- l'extrémité côté distal du conduit d'apport rapporté présente la forme d'un biseau,
- l'extrémité côté distal du conduit d'apport rapporté se trouve au voisinage des moyens de déflexion,
- le conduit d'apport rapporté comporte une pluralité d'orifices d'apports distaux indépendants,
- le conduit d'apport rapporté comporte une pluralité de canaux indépendants,
et/ou, dans certaines variantes :
- un conduit d'alimentation de gaz relie le/les canaux auxiliaires du dispositif à/aux sources de gaz respirables, ledit conduit d'alimentation de gaz comportant du côté de la/des sources, un dispositif à perte de charge apte à limiter le débit et la pression dudit gaz respirable disponible à la sortie de ladite source et à imposer audit jet de gaz respirable une valeur de débit et une valeur de pression prédéterminées et, du côté du/des canaux auxiliaires, comporte une soupape d'échappement tarée apte à mettre ledit conduit d'alimentation de gaz en communication avec l'atmosphère dès que la pression dans ledit conduit d'alimentation de gaz dépasse ladite valeur de pression prédéterminée,
- le dispositif à perte de charge est réglable de façon à permettre d'imposer aux jets de gaz respirable une pluralité de valeurs de débit et de valeurs de pression prédéterminées ou préréglées,
- le tarage de la soupape d'échappement tarée est réglable,
- le dispositif à perte de charge est incorporé à l'élément tubulaire,
- le dispositif à perte de charge est extérieur à l'élément tubulaire,
- la soupape d'échappement tarée est incorporée à l'élément tubulaire,
- la soupape d'échappement tarée est extérieure à l'élément tubulaire,
- le dispositif comporte un humidificateur dans le conduit d'alimentation de gaz reliant la/les sources de gaz respirables à/aux canaux auxiliaires,
- l'humidificateur est disposé entre le dispositif à perte de charge et la soupape d'échappement tarée,
et/ou, dans certaines variantes :
- le dispositif comporte une vanne commandée susceptible d'obturer au moins partiellement l'extrémité proximale du canal principal au moins pendant l'insufflation du gaz respirable,
- la vanne commandée forme un ensemble monolithique avec l'élément tubulaire,
- la vanne commandée est rapportée sur l'élément tubulaire,
- la vanne commandée est solidaire d'un embout susceptible d'être emboîté sur l'extrémité proximale de l'élément tubulaire,
- la vanne commandée comporte une enceinte étanche à section toroïdale, disposée au voisinage de l'extrémité proximale du canal principal et comportant au moins une paroi interne souple et élastique qui, en se dilatant ou en se rétractant en réponse à l'introduction ou à l'évacuation d'un fluide de gonflage dans ladite enceinte étanche, commande la section de passage dudit canal principal,
- le bord libre de l'extrémité proximale de l'élément tubulaire comporte au moins une échancrure,
- le dispositif comporte au moins une prise de pression disposée du côté de l'extrémité distale de l'élément tubulaire,
- la prise de pression comporte une chambre périphérique annulaire coaxiale à l'élément tubulaire et débouchant côté distal du dispositif par un passage annulaire distal, ladite chambre périphérique annulaire étant en relation avec un embout de sortie latéral,
- un filtre, fibreux ou poreux annulaire est disposé dans la chambre périphérique annulaire,
- la prise de pression est formée par l'orifice de sortie par lequel un des canaux auxiliaires ménagés dans la paroi de l'élément tubulaire débouche au voisinage de l'extrémité distale dudit élément tubulaire,
- le dispositif comporte des moyens recevant la pression prélevée par ladite prise de pression et susceptibles d'imposer l'ouverture à la vanne commandée pour dégager le canal principal,
- le dispositif comporte une vanne d'échappement tarée disposée à l'extrémité proximale de l'élément tubulaire, du côté opposé au bord libre de l'extrémité proximale par rapport à ladite vanne commandée,
- l'introduction et l'évacuation du fluide de gonflage dans/de ladite enceinte étanche résulte du déplacement bidirectionnel d'un volume de fluide approprié contenu dans une capacité tampon à volume variable commandable,
- le fluide de gonflage est un gaz.

L'invention concerne également un appareil nasal d'assistance respiratoire selon la revendication 13.

Dans divers modes de mise en oeuvre de l'appareil, les moyens suivants pouvant être utilisés seuls ou selon toutes les combinaisons techniquement possibles, sont employés :
- le dispositif incorporé à l'appareil présente une ou plusieurs des caractéristiques décrites concernant ledit dispositif,
- l'appareil comporte une pièce monolithique incorporant deux dispositifs selon l'invention,
- dans la pièce monolithique les deux éléments tubulaires sont parallèles entres eux,
- l'appareil comporte en outre un moyen d'apport de liquide à nébuliser dans le flux gazeux traversant l'appareil vers le patient,
- l'appareil comporte en relation avec chaque élément tubulaire deux parties de diamètres différents disposées en série, la partie de plus grand diamètre étant proximale, c'est-à-dire du côté de l'air libre opposé au côté du patient, et étant pourvue d'un raccord tubulaire latéral,
- les parties de plus grand et de plus petit diamètres sont séparées par une partie intermédiaire conique,
- l'appareil comporte, pour chaque partie de plus grand diamètre un manchon disposé dans ladite partie de plus grand diamètre et y ménageant un espace de section annulaire, le raccord tubulaire latéral débouchant dans ledit espace de section annulaire, des moyens de solidarisation du manchon à ladite partie de plus grand diamètre obturant ledit espace de section annulaire du côté proximal opposé à une partie intermédiaire conique reliant la partie de plus grand diamètre proximale de la partie de plus petit diamètre,
- l'appareil comporte pour chaque partie intermédiaire conique un conduit latéral d'apport de liquide débouchant dans ladite partie intermédiaire conique, au voisinage du raccord de celle-ci et de la partie de plus petit diamètre,
- chaque conduit latéral d'apport fait saillie à l'intérieur de ladite partie intermédiaire conique et présente un biseau dont l'inclinaison est opposée à celle de ladite partie intermédiaire conique, à l'endroit où débouche ledit conduit latéral,
- l'appareil est pourvu d'un dispositif d'alimentation en gaz respirable(s), associé à une source d'un/de tel(s) gaz et pourvu d'un détecteur de pression apte à détecter le début des expirations et le début des inspirations du patient ventilé, ledit dispositif d'alimentation comportant en outre, alimentés en parallèle par ladite source, des moyens de calibrage délivrant en permanence un courant de gaz respirable(s) sous une première pression apte à maintenir ouvertes les bronchioles dudit patient en évitant le collapsus et une vanne commandable calibrée pour délivrer, lorsqu'elle est ouverte, un courant de gaz respirable(s) sous une seconde pression, supérieure à ladite première pression et apte à assister efficacement l'inspiration dudit patient, ladite vanne commandable étant commandée par ledit détecteur de pression, à la fermeture pendant les expirations et à l'ouverture pendant les inspirations,
- le dispositif d'alimentation comporte un débitmètre interposé entre les moyens de calibrage et la vanne commandable, d'une part, et les canaux auxiliaires, d'autre part,
- le dispositif d'alimentation comporte un dispositif d'alarme commandé par le détecteur de pression,
- une prise de pression pour le détecteur débouche dans au moins l'un des éléments tubulaires en aval, côté distal, c'est-à-dire côté patient, desdits moyens de déflexion et au voisinage de l'extrémité distale dudit élément tubulaire, ladite prise de pression étant reliée au détecteur par un tube de raccordement,
- la prise de pression est un trou traversant la paroi de l'élément tubulaire et le tube de raccordement est extérieur à ce dernier,
- le tube de raccordement est disposé à l'intérieur de l'élément tubulaire et la prise de pression est formée par une échancrure latérale, pratiquée dans le tube de raccordement et ayant une longueur égale à plusieurs fois le diamètre dudit tube de raccordement,
- l'appareil comporte des organes mobiles aptes à restreindre la section de passage des jets gazeux au niveau des moyens de déflexion,
- les organes mobiles sont constitués par les extrémités distales de fourreaux pouvant glisser à frottement dur à l'intérieur des canaux principaux,
- l'appareil comporte, sur chacune de ses deux extrémités distales, un manchon de narine, souple, rapporté extérieurement, et destiné à venir au contact de la paroi interne de la narine correspondante,
- chaque manchon de narine comporte une ouverture en regard de l'ouverture distale de l'élément tubulaire,
- chaque manchon de narine est en une matière mousse à cellules ouvertes et obture l'ouverture distale de l'extrémité distale de l'élément tubulaire, tout en assurant le passage aux gaz respirable(s) et aux éventuels liquides nébulisés.

L'invention concerne également un masque d'assistance respiratoire muni d'un dispositif selon l'invention. Le dispositif en relation avec le masque présente une ou plusieurs des caractéristiques décrites concernent ledit dispositif.

La présente invention va maintenant être exemplifiée sans pour autant en être limitée avec la description qui suit en relation avec les figures suivantes:
- la Figure 1 est une vue schématique et partielle, en coupe axiale agrandie, d'un premier mode de réalisation du dispositif de l'invention,
- les Figures 2 et 3 sont des coupes transversales, respectivement selon les lignes II-II et III-III de la figure 1,
- les Figures 4 et 5 illustrent, schématiquement en coupe axiale agrandie, deux variantes de réalisation pour l'extrémité distale du dispositif selon l'invention,
- la Figure 6 montre, en coupe axiale schématique et partielle, une variante à double flux opposés du dispositif selon la présente invention,
- la figure 7 montre, en coupe axiale, une variante de réalisation du dispositif de l'invention,
- les figures 8, 9 et 10 sont des coupes transversales du dispositif de la figure 7, respectivement selon les lignes III-III, IV-IV et V-V,
- la figure 11 montre, en coupe axiale, une vue schématique d'une partie du dispositif de l'invention selon un premier mode de réalisation de tube intérieur brise jet et de dérivation, et
- les Figures 12 et 13 montrent deux vues d'un autre exemple de réalisation de l'invention.

Dans son principe, le dispositif de l'invention est donc constitué d'un élément tubulaire, en général un tube, qui forme un canal principal et qui est destiné à être relié par son extrémité distale à la voie respiratoire du patient alors que l'extrémité proximale de l'élément tubulaire débouche à l'air libre à l'extérieur du patient, le système respiratoire du patient étant donc relié à l'extérieur par l'intermédiaire du canal principal du dispositif. Des canaux auxiliaires sont pratiqués dans l'épaisseur de l'élément tubulaire et débouchent dans le canal principal. Les canaux auxiliaires sont alimentés en gaz respiratoire(s) par l'intermédiaire d'un conduit d'alimentation. Le dispositif comporte des moyens de déflexion pour faire converger les uns vers les autres, à l'intérieur du canal principal, les jets de gaz respiratoire(s) injectés par lesdits canaux auxiliaires vers le canal principal. De préférence, les moyens de déflexion des jets de gaz respirable(s) dévient les jets vers l'axe central du canal principal.

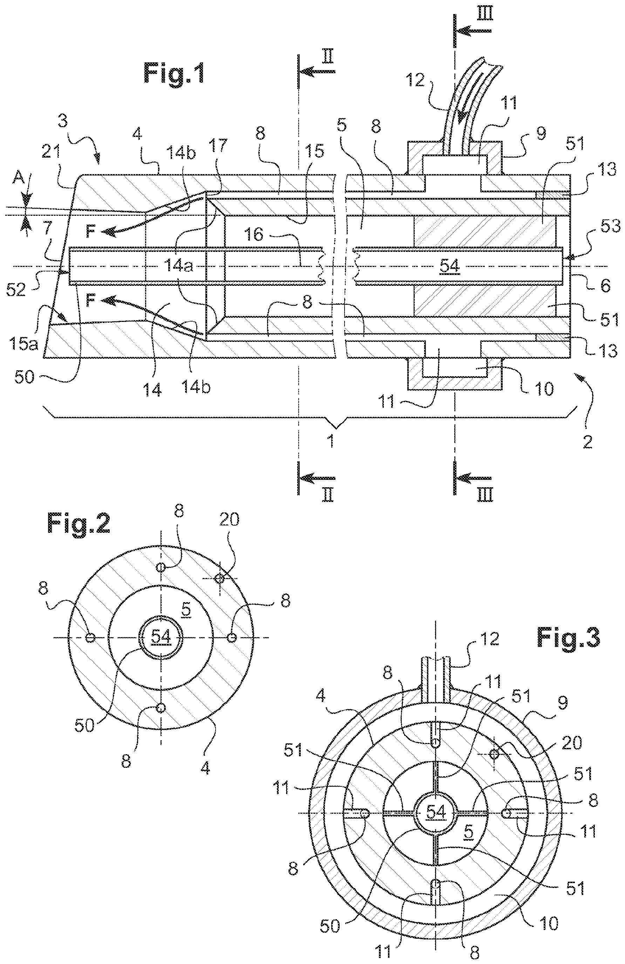

Sur la Figure 1, on a représenté schématiquement et à grande échelle, l'élément tubulaire avec ses extrémités proximale 2 et distale 3 d'un mode de réalisation 1 du dispositif selon l'invention. Ce mode de réalisation peut constituer ou être combiné à, par exemple, un masque facial, un masque laryngé, un masque pharyngé, un adaptateur nasal, une sonde endotrachéale, oro-nasale avec ou sans ballonnet, une sonde endotrachéale pédiatrique, une sonde de monitorage des gaz, une sonde endobronchique, une sonde nasopharyngée, une sonde d'intubation anatomique pour enfant, une sonde de Cole néonatale, une sonde canule de Guedel, ou une sonde nasale d'oxygénothérapie. Le dispositif de l'invention peut être utilisé en toute position, extracorporelle ou intracorporelle et notamment supraglottique ou supratrachéale.

En fait, le domaine d'application de l'invention est très large : ventilation invasive ou non invasive, adulte ou pédiatrique, notamment : valve CPAP type Boussignac, CPAP type monojet, CPAP spécifique à l'utilisation pour apnée du sommeil, valve pour Arrêt cardiaque et en tant qu'interface variée : masque / masque laryngé / lunette à oxygène / sonde, etc.

Le dispositif 1 comporte un élément tubulaire 4 souple, notamment pour s'adapter à la morphologie du patient, délimitant un canal principal 5 débouchant, par l'orifice proximal 6, à l'extrémité proximale 2, et, par l'orifice distal 7, à l'extrémité distale 3. Dans une variante, l'élément tubulaire 4 est préformé et rigide.

Ainsi, le canal principal 5 est capable d'assurer le passage entre les orifices 6 et 7, dont l'un, l'orifice distal 7, est destiné à se trouver à l'intérieur des voies respiratoires d'un patient ou à l'extérieur en relation avec ces dernières et l'autre, l'orifice proximal 6, est destiné à se trouver en communication avec l'environnement extérieur dudit patient. Cet orifice proximal 6 peut déboucher à l'air libre et, dans ce cas, le patient peut inspirer de l'air frais et expirer l'air vicié à travers le canal principal 5. On peut également relier l'orifice proximal 6 à au moins une source de gaz respirable(s) sous pression (non représentée) et prévoir un système de valves unidirectionnelles, pour que le patient inspire le(les) gaz respirable de ladite source à travers ledit canal principal 5 et expire le gaz vicié à l'air libre, également à travers ce canal principal 5.

Le diamètre du canal principal 5 est de l'ordre de quelques millimètres. Des essais satisfaisants ont été effectués avec des diamètres de 3 mm, 7 mm et 8 mm, voir un peu plus.

Par ailleurs, dans l'épaisseur de la paroi de l'élément tubulaire 4 sont ménagés des canaux auxiliaires 8, s'étendant sur la presque totalité de la longueur du canal principal 5. Ces canaux auxiliaires 8 sont destinés à être reliés à une (ou plusieurs) source de gaz respirable sous pression (non représentée). Par exemple, cette pression est de quelques bars, par exemple 1,2 ou 4 bars, et elle est réglable. De préférence, comme représentés, ces canaux auxiliaires sont des canaux individualisés sur au moins une partie de leurs trajets. Dans des variantes, les canaux auxiliaires ont une partie commune formant une chambre circulaire coaxiale et extérieure au canal principal ou sont réduits pour ce qui concerne leur trajet individuel aux orifices de sortie débouchant dans le canal principal et, dans ce dernier cas, on peut considérer qu'il n'y a qu'un canal auxiliaire. Dans tous les cas, le/les canaux auxiliaires débouchent dans le canal principal par plusieurs orifices de sortie pour y former des jets de gaz respirable(s).

Comme cela est représenté sur les Figures 1 et 3, la liaison à la ou aux sources de gaz respirables sous pression peut être réalisée au moyen d'une bague 9, entourant de façon étanche l'élément tubulaire 4, du côté de l'extrémité proximale 2, et délimitant une chambre annulaire étanche 10 autour dudit élément tubulaire. Les canaux auxiliaires 8 sont mis en communication avec la chambre 10 grâce à des arrachements locaux 11 de la paroi de l'élément tubulaire 4 et ladite chambre 10 est reliée à/aux sources de gaz respirable(s) par une liaison 12. Bien entendu, les extrémités proximales des canaux auxiliaires 8 sont obturées, par exemple par des bouchons 13. Les canaux auxiliaires 8 ont un diamètre plus petit que celui du canal principal 5.

Du côté distal, chacun des canaux auxiliaires 8 débouche par un orifice de sortie respectif 17 dans un évidement 14 de la paroi interne 15 de l'élément tubulaire 4. L'évidement 14 est annulaire et centré sur l'axe 16 central de révolution de l'élément tubulaire côté de l'extrémité distale 3. Il comporte une face 14a, sensiblement transversale ou légèrement inclinée de façon à constituer un évasement du canal principal 5, dans laquelle débouchent lesdits canaux auxiliaires 8 par leurs orifices de sortie17, ainsi qu'une face 14b suivant la face 14a et convergeant en direction de l'axe 16.

De préférence, entre la face inclinée convergente 14b et l'orifice distal 7, la paroi interne 15 présente une partie 15a légèrement évasée vers l'extérieur, comme cela est illustré par l'angle A sur la Figure 1.

Ainsi, lorsque les canaux auxiliaires 8 sont alimentés en gaz respirable(s) sous pression à travers les éléments 9 à 12, les jets gazeux correspondants heurtent la face inclinée 14b, qui les défléchit en direction de l'axe 16 (flèches F sur la Figure 1), formant ainsi des moyens de déflexion, et engendrant au voisinage de celui-ci une zone de dépression favorisant la circulation gazeuse à l'intérieur du canal principal 5, de l'orifice proximal 6 vers l'orifice distal 7. On favorise ainsi l'inspiration du patient.

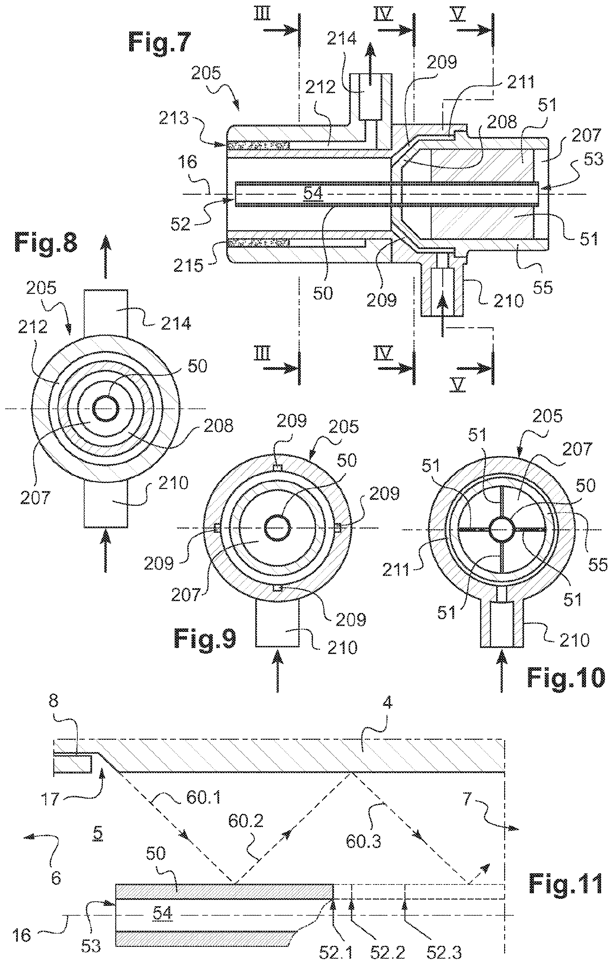

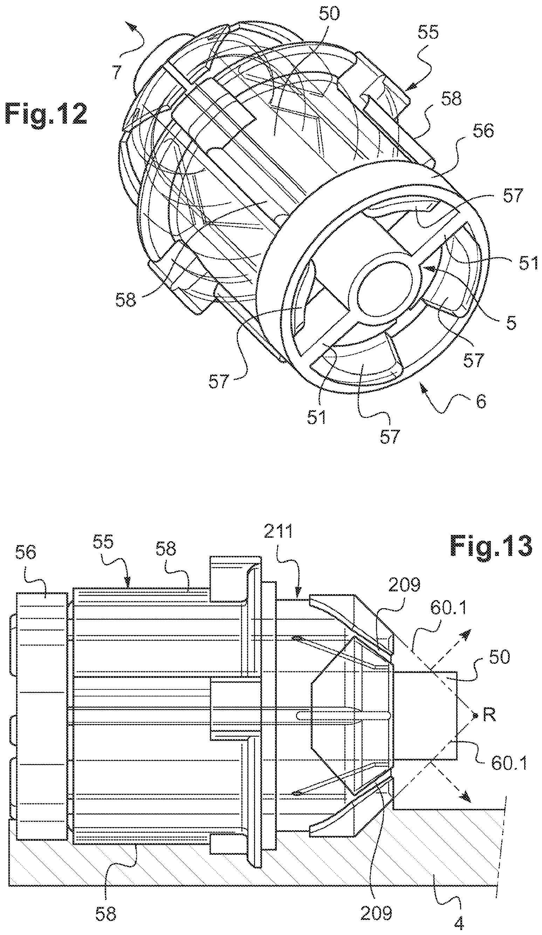

On comprend qu'il est possible de réaliser les canaux auxiliaires au sein du dispositif de manière différente de celle représentée à titre d'exemple. Ainsi, par exemple, les canaux auxiliaires peuvent être des arrachements 11 ou gorges sur toutes leurs longueurs plutôt que de se poursuivre en des canaux noyés dans la paroi de l'élément tubulaire comme représentés, lesdits arrachements étant fermés extérieurement par une paroi externe tubulaire rapportée s'appliquant sur l'élément tubulaire. Ladite paroi externe tubulaire peut également former au niveau des orifices de sortie des canaux auxiliaires, les moyens de déflexion. Lesdits canaux auxiliaires peuvent également être réalisés entre la paroi interne 15 de l'élément tubulaire 4 et la paroi externe d'un corps tubulaire (référencé 55 Figures 7 et 10), une pièce monobloc à tube intérieur et ailettes s'insérant en outre dans le corps tubulaire 55, l'élément tubulaire 4 et/ou le corps tubulaire 55 comportant des éléments structuraux adaptés à cette fin. En variante, un anneau circulaire fait partie de la pièce monobloc et c'est cet anneau circulaire qui est inséré directement dans l'élément tubulaire, l'anneau circulaire faisant alors office de corps tubulaire. Dans d'autres variantes à pièce monobloc à anneau circulaire, l'anneau circulaire de la pièce monobloc est inséré dans le corps tubulaire, ce dernier étant inséré dans l'élément tubulaire (Voir Figures 12, 13).

On comprend que l'on met en oeuvre un anneau circulaire, c'est-à-dire qui forme un cercle complet essentiellement pour des raisons de résistance mécanique. Si la matière de la pièce monobloc est suffisamment résistante, on peut envisager que l'anneau en question soit discontinu en arcs aux extrémités périphériques des ailettes.

De préférence, la distance entre chacun des orifices de sortie 17 des canaux auxiliaires 8 et l'orifice distal 7 est de l'ordre de 1 à 2 cm.

En aval de l'orifice distal 7, la pression dans la cavité pulmonaire est faible et pratiquement constante.

En variante, les orifices distaux des canaux auxiliaires formant les jets sont des orifices en bandes semi-annulaires, voire un seul orifice en bande annulaire ou quasi annulaire, au lieu de ponctuels. Dans le cas d'un seul orifice en bande annulaire, on comprend qu'il peut être en pratique quasi annulaire, quelques ponts de matière joignant les deux bords de l'orifice de sortie en bande afin de mieux les maintenir à une distance déterminée entre eux, distance qui correspond à l'épaisseur de l'orifice en bande. De préférence les orifices ponctuels ont un diamètre réduit et les orifices en bandes une épaisseur réduite, typiquement moins de 150 µm comme mentionné dans la demande de brevet PCT/FR2013 051979.

Un tube intérieur 50 est disposé centralement dans le canal principal. Le tube intérieur 50 est coaxial, le long de l'axe 16, au canal principal 5. Le tube intérieur 50 est creux définissant une lumière 54 et ouvert à ses deux extrémités : une extrémité distale 52 et une extrémité proximale 53 qui sont orientées vers les extrémités et ouvertures correspondantes de même qualificatif de l'élément tubulaire 4. Le tube intérieur 50 est maintenu en position grâce à des ailettes 51 étendues entre le tube intérieur et la paroi interne 15 de l'élément tubulaire 4. Dans une variante non représentée sur la Figure 1, un anneau circulaire (représenté en 56 Figures 12, 13) est interposé entre la paroi interne 15 de l'élément tubulaire 4 et les ailettes 51, ces dernières étant fixées sur ledit anneau circulaire. Les ailettes ont une épaisseur réduite afin de ne pas entraîner, à leur niveau, une réduction trop importante de l'espace de circulation des gaz dans le canal principal 5. Leur nombre est également réduit, compris entre une et quatre de préférence. Sur les figures 1 à 10, quatre ailettes sont représentées. Les ailettes sont disposées radialement autour du tube intérieur 50 et équiangulairement de préférence, soit pour quatre ailettes à 90° l'une de l'autre autour du tube intérieur. Le tube intérieur est sensiblement rigide. On peut envisager qu'il soit souple mais dans ce cas, il faut que les ailettes s'étendent le long du tube intérieur afin que ce dernier suive au mieux les déformations de l'élément tubulaire 4 au cas où celui-ci serait aussi souple. De préférence, on ne dispose pas d'ailettes dans la zone de production des jets, c'est-à-dire la zone des orifices de sortie des canaux auxiliaires

On comprend donc que les ailettes, ici disposées côté de l'extrémité proximale de l'élément tubulaire, peuvent être disposées en d'autres positions et/ou être plus ou moins allongées et/ou réparties en plusieurs ensembles d'ailettes sur la longueur du canal principal.

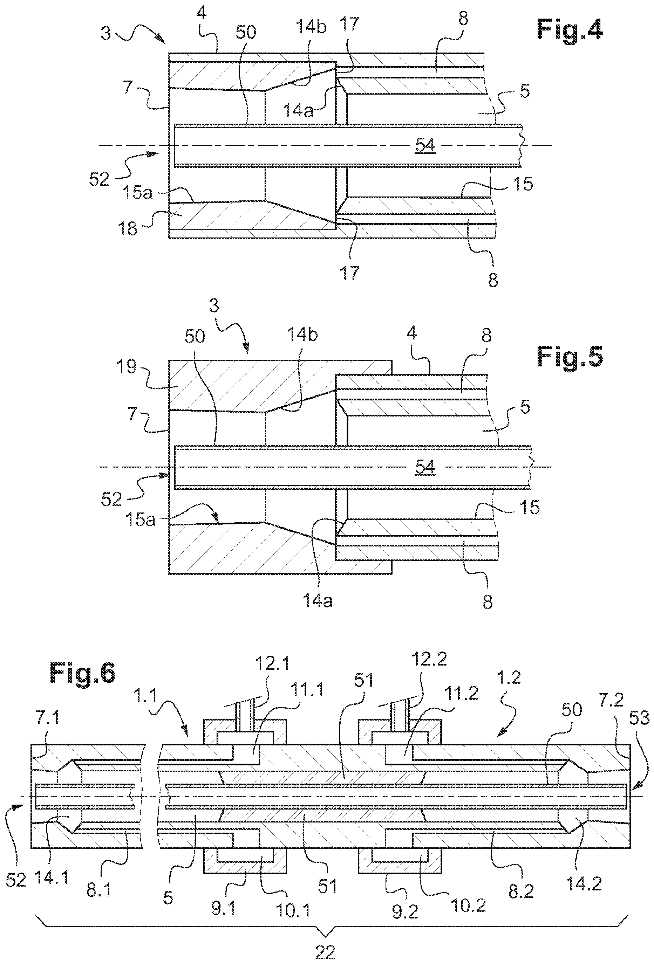

Dans le mode de réalisation de l'invention illustré par la Figure 1, on a indiqué que l'ensemble des faces 14a et 14b était réalisé par évidement 14 de la paroi interne 15 du canal principal 5. Il va de soi que ce mode de réalisation n'est pas limitatif et que les faces 14a et 14b peuvent être obtenues de façons différentes. Par exemple, sur les Figures 4 et 5, la face 14a est formée dans la paroi interne 15 de l'élément tubulaire 4, alors que la face 14b est prévu sur un embout 18 ou 19 venant s'emboîter intérieurement, embout 18, ou extérieurement, embout 19, sur l'élément tubulaire 4, et dans ce cas, l'orifice distal 7 et la paroi divergente 15a sont portés par l'embout 18 ou 19 correspondant.

Dans une variante de réalisation de l'évidement 14, les moyens de déflexion ne forment pas une gorge annulaire continue mais sont constitués d'un ensemble discontinu d'évidements de forme générale conique, ménagés dans la paroi interne, et au fond de chacun desquels débouche par son orifice de sortie l'extrémité distale d'un canal auxiliaire. Le principe de fonctionnement d'un tel dispositif est bien entendu comparable à celui décrit plus haut.

Comme le montrent les Figures 2 et 3, les canaux auxiliaires 8 sont disposés régulièrement autour de l'axe de l'élément tubulaire 4. Leur nombre est variable suivant les utilisations (adulte ou enfant), mais il est généralement compris entre un et dix. D'une manière générale, le nombre des orifices de sortie du/des canaux auxiliaires et le diamètre des orifices de sortie et/ou des canaux auxiliaires sont ajustés afin de permettre un débit suffisant au(x) gaz respirable(s). On peut, à ce sujet, relever l'intérêt des orifices de sortie en forme de bande annulaire ou semi annulaire qui permettent d'avoir une surface de sortie relativement importante malgré une épaisseur qui peut être faible par rapport à des orifices ponctuels de faible diamètre qui seraient multipliés.

On peut également voir sur ces Figures 2 et 3 que le tube intérieur 50 avec sa lumière interne 54 est disposé centralement dans le canal principal 5 de l'élément tubulaire 4. Dans des variantes non représentées, le tube intérieur, tout en étant parallèle à l'axe principal du canal principal, est décalé radialement par rapport à cet axe du canal principal. Le tube intérieur 50 est maintenu en place par des ailettes 51 qui sont vues en coupe transversale sur la Figure 3. On a mis en oeuvre dans cet exemple quatre ailettes 51 disposées radialement équiangulairement à 90° l'une de la suivante autour du tube intérieur 50. Les ailettes s'appliquent directement sur la surface interne de l'élément tubulaire dans cet exemple. Des guides en creux d'insertion et guidage des ailettes peuvent être réalisés dans l'épaisseur de la surface interne de l'élément tubulaire pour faciliter l'insertion du tube intérieur et de ses ailettes. De préférence, le tube intérieur et ses ailettes forment une pièce monobloc. Dans des variantes, la pièce monobloc comporte en outre un anneau circulaire auquel les ailettes sont reliées et c'est cet anneau circulaire qui s'applique par sa surface extérieure sur la surface interne de l'élément tubulaire. On peut prévoir un ergot externe sur l'anneau circulaire et un guide creux de la surface interne de l'élément tubulaire _ou d'un corps tubulaire s'insérant dans l'élément tubulaire_, qui sont complémentaires, pour faciliter l'insertion de la pièce monobloc qui comporte cette fois en plus l'anneau circulaire. Un moyen de verrouillage entre la pièce monobloc et l'élément tubulaire ou le corps tubulaire peut être prévu.

On peut noter que la présence d'ailettes 51 dans l'extrémité proximale de l'élément tubulaire forme un obstacle empêchant l'introduction accidentelle d'un objet pouvant obturer hermétiquement ladite extrémité proximale.

Dans une variante, le tube intérieur est un élément individuel et des supports à ailettes en forme de roue servent à maintenir le tube centralement. Les supports à ailettes comportent vers leur centre un moyeu annulaire dans lequel le tube est maintenu et à leur périphérie un anneau circulaire du type mentionné précédemment. Ces supports à ailettes ont une longueur (dans la direction de l'axe 16 de l'élément tubulaire) réduite par rapport à ceux décrits précédemment et on peut en prévoir plusieurs à l'intérieur du canal principal, par exemple un côté proximal, un médian et un côté distal de l'élément tubulaire pour un tube allongé de longueur voisine de l'élément tubulaire. Dans une variante, les supports à ailettes forment avec le tube intérieur une pièce monobloc et dans ce cas le moyeu annulaire peut être omis, les ailettes venant directement sur le tube intérieur.

Sur les Figures 1 à 5, le tube intérieur s'étend continu entre pratiquement les deux extrémités proximale et distale de l'élément tubulaire. On comprend cependant que le tube intérieur peut être moins long que la longueur de l'élément tubulaire mais dans ce cas il doit être présent au niveau des jets de gaz défléchis produit par les orifices de sortie des canaux auxiliaires afin que son rôle de brise jet et de dérivation soit effectif. On rappelle que le tube intérieur est creux et ouvert à ses deux extrémités, il sert donc également de dérivation notamment pour les gaz expirés par le patient. Il en résulte, en particulier en cas d'hémoptysie, que le sang expiré peut passer par la lumière 54 du tube intérieur 50, ce qui évite qu'il soit nébulisé ou pulvérisé dans les jets défléchis.

On remarquera qu'au moins l'un des canaux auxiliaires 8, au lieu d'être relié en commun avec les autres à la source de gaz respirable sous pression (par l'intermédiaire des éléments 9 à 12) peut être continuellement alimenté par la source de gaz respirable, de façon à maintenir une pression positive dans les poumons du patient et cela pendant ou à la fin de la phase expiratoire provoquée par l'insufflation de gaz dans les canaux auxiliaires 8 (effet anti-collapse).

Selon encore une autre variante de l'invention, un des canaux auxiliaires 8 peut également être spécialisé pour apporter un fluide médical ou un fluide d'humidification, si la source sous pression ne présente pas les caractéristiques requises.

Pour assurer l'humidification, le canal auxiliaire apportant de l'eau, de préférence tiède, est de préférence recourbé en forme de U à son extrémité distale, et vient déboucher dans une cavité ménagée dans la paroi interne 15, cavité dans laquelle débouche également un canal apportant de l'air sous pression. Dans ladite cavité, située de préférence entre l'évidement 14 et l'orifice distal 7, le canal d'eau et le canal d'air débouchent vis-à-vis l'un de l'autre, c'est-à-dire sensiblement sur le même axe, les deux fluides, air et eau, arrivant en sens contraires, ce qui permet la vaporisation de l'eau, la vapeur obtenue étant ensuite entraînée par l'air insufflé.

Au moins un canal supplémentaire 20 peut être prévu dans l'épaisseur de l'élément tubulaire 4 afin de déboucher dans la face d'extrémité distale 21 de l'élément tubulaire 4 et servir de logement à un dispositif de mesure de pression (non représenté).

Lorsqu'au moins deux prises de pression sont présentes, notamment à chacune des extrémités de l'élément tubulaire, elles permettent, grâce à la différence des pressions mesurées, de calculer le débit gazeux.

L'élément tubulaire 4 peut comporter à l'extrémité distale 3, un ballonnet gonflable (non représenté) muni des dispositifs nécessaires de sécurité ou tout autre ballonnet permettant de se comporter comme une soupape de sécurité en cas de surpression dans les poumons. Cet éventuel ballonnet peut être gonflé à partir d'un canal supplémentaire (non représenté) associé à l'élément tubulaire 4.

D'autres moyens de sécurités destinés à éviter une surpression préjudiciable dans la voie aérienne du patient peuvent être mis en oeuvre, notamment des moyens de régulation du débit ou de la pression des gaz respirables destinés à être injectés dans le canal principal du dispositif d'assistance respiratoire. Ces moyens sont une ou des vannes commandées et des dispositifs de mesure de pression, de calcul décisionnel et de commande de vannes. En outre, il est également possible de mettre en oeuvre des moyens d'introduction d'air de l'environnement. En fait, le principe de l'invention qui consiste à utiliser un tube brise jet au sein d'un dispositif à jets défléchis de type CPAP BOUSSIGNAC peut être mis en oeuvre dans toutes les applications des dispositifs à jets défléchis de type CPAP BOUSSIGNAC.

Un dispositif de sécurité peut être constitué simplement d'un manchon élastique entourant l'élément tubulaire, partiellement collé à ce dernier, et recouvrant une perforation pratiquée à travers la paroi dudit élément tubulaire, notamment au voisinage de l'extrémité proximale. Ainsi, lorsque la pression interne devient trop élevée, le gaz peut s'écouler à travers ladite perforation, puis entre la paroi externe de l'élément tubulaire et la paroi interne du manchon élastique. Si un manchon de sécurité est également prévu au voisinage de l'extrémité distale, la perforation correspondante, étant placée au-delà d'un éventuel ballonnet de maintien par friction, doit mettre en communication l'intérieur de l'élément tubulaire avec l'air ambiant ; ledit ballonnet doit donc être contourné, ce que l'on obtient par exemple en le plaçant autour du manchon élastique.

La variante de réalisation 22 double flux opposés du dispositif selon l'invention, montrée par la Figure 6, comporte deux dispositifs 1.1 et 1.2, chacun de structure semblable à celui de la Figure 1, inversés, accolés par leurs extrémités proximales, le dispositif 1.2 pouvant être plus court que le dispositif 1.1. Dans cette variante de réalisation 22, l'orifice distal (côté patient) du dispositif est constitué par l'orifice distal 7.1 du dispositif 1.1, tandis que l'orifice proximal (côté opposé au patient) est formé par l'orifice 7.2 du dispositif 1.2. Chacun des dispositifs 1.1 et 1.2 est pourvu de son système d'alimentation 9.1, 9.2, 10.1, 10.2, 11.1, 11.2 et 12.1, 12.2 en gaz respirable sous pression, alimentant des canaux respectifs 8.1 ou 8.2, débouchant dans des évidements annulaires 14.1 ou 14.2, proches respectivement desdits orifices 7.1 et 7.2. Le dispositif 22 forme une sonde à double flux. Ce dispositif étant en place sur le patient, c'est-à-dire le dispositif 1.1 étant relié à la voie respiratoire de celui-ci, le dispositif 1.2 et les bagues 12.1 et 12.2 étant extérieures au patient, on alimente alternativement les canaux 8.1 à travers les éléments 9.1 à 12.1 et les canaux 8.2 à travers les éléments 9.2 à 12.2 en gaz respirable(s) sous pression, de manière à favoriser alternativement l'inspiration et l'expiration du patient.

A cet effet, un dispositif (non représenté) de commutation et de réglage des débits et des durées d'insufflation de gaz est relié aux bagues 9.1 et 9.2 d'une part, et à la source de gaz d'autre part.

L'élément tubulaire respiratoire ainsi constitué permet une assistance respiratoire en insufflation et une assistance respiratoire en expiration, à condition de maintenir un débit continu assurant la sécurité et limitant le risque de collapsus pulmonaire.

La pression dans les canaux 8.2 est avantageusement plus élevée que la pression dans les canaux 8.1, car l'effet d'entraînement du fluide contenu dans la sonde n'est pas de même nature.

Cette variante 22 double flux opposés comporte un seul tube intérieur 50 commun aux deux dispositifs à jets défléchis montés tête-bêche. Les ailettes de maintien 51 sont cette fois disposées vers le milieu du canal principal, entre les orifices de sortie des canaux auxiliaires des deux dispositifs à jets défléchis. Le tube intérieur s'étend sur sensiblement toute la longueur du canal principal de cette variante, son extrémité distale 52 et son extrémité proximale 53 venant pratiquement au niveau des extrémités correspondantes de l'élément tubulaire. Dans une variante, on prévoit deux tubes intérieurs, un pour chaque dispositif à jets défléchis, chacun étant présents au moins dans la zone des jets défléchis correspondante afin de former le brise jet et la dérivation prévue.

A noter que les Figures de la présente demande ne sont pas à l'échelle et que certaines des parties des éléments représentés peuvent être plus ou moins agrandies/allongées ou rétrécies/raccourcies selon les besoins. En particulier, côté distal, on s'assure de préférence que le tube intérieur 50 tout comme l'élément tubulaire se termine par son extrémité distale 52 à un niveau où les jets ont disparus ou, à tout le moins, en dehors d'une zone de turbulence forte ou de présence de jet(s). En effet, c'est la puissance des jets et les turbulences créées par ces jets qui sont cause d'une possible nébulisation ou pulvérisation. En fait, la rencontre des jets défléchis sur le tube brise jet 50 provoque également des turbulences qui finissent par s'atténuer rapidement vers l'extrémité distale de l'élément tubulaire.

Le dispositif de l'invention peut donc être destiné à être introduit dans ou relié à une voie respiratoire d'un patient. Il peut, dans d'autres applications, constituer l'embout d'un masque. Selon les diverses applications possibles et le type de patient (adulte, enfant...), les dimensions, notamment longueur et diamètres, seront différentes.

Ainsi, l'élément tubulaire peut constituer l'embout d'entrée et de sortie d'air d'un masque qui, par ailleurs, est de type connu, c'est-à-dire comprenant essentiellement une coque, un bourrelet d'étanchéité et des moyens de fixation tels que des sangles. L'élément tubulaire utilisé pour le masque peut être du type où l'assistance respiratoire n'agit que dans le sens de l'inspiration (comme dans le cas du dispositif de la Figure 1) mais, dans une variante, le masque peut être équipé d'un dispositif double flux opposés selon la Figure 6, pour l'assistance à l'inspiration et à l'expiration.

En général, un masque respiratoire facial comporte une coque rigide de forme générale tronconique, pouvant être appliquée sur le visage d'un patient par l'intermédiaire d'un coussinet bordant son ouverture périphérique. Du côté opposé au visage, le masque est pourvu d'un dispositif d'assistance respiratoire conforme à la présente invention, comportant un élément tubulaire solidaire de ladite coque ou emboîté sur une saillie tubulaire de celle-ci. L'élément tubulaire, par exemple 205 des Figures 7 à 10, sert d'embout d'entrée et de sortie de gaz dans le masque, son extrémité proximale, opposée au patient, étant à l'air libre, alors que son extrémité distale, côté patient, se raccorde au masque. Le gaz respiratoire est amené dans une chambre annulaire périphérique et aux canaux auxiliaires périphériques par un conduit d'alimentation relié, d'un côté, à un embout d'amenée du dispositif et, de l'autre, à une source de gaz respiratoire(s), telle qu'une bouteille de gaz sous pression. On peut prévoir à l'intérieur du conduit d'alimentation un bouchon percé d'un passage longitudinal permettant le réglage de la pression des gaz dans le dispositif. Par exemple, le bouchon peut être obtenu par découpe d'un tronçon sur la longueur d'un profilé dont le passage longitudinal présente un diamètre d constant. On comprendra aisément qu'en faisant varier la longueur du bouchon et de son tronçon, on fait varier la perte de charge apportée par ce dernier dans le conduit d'alimentation. On peut ainsi, par expérimentation, régler ladite perte de charge pour que, en utilisation, la pression du gaz respiratoire à l'extrémité distale de l'élément tubulaire corresponde à une oxygénation optimale et sans danger, du patient. Par ailleurs, l'élément tubulaire comporte un embout pour le prélèvement de gaz et/ou la mesure de pression.

Le dispositif d'assistance respiratoire de l'invention peut, dans d'autres applications, se présenter sous forme d'un appareil nasal d'assistance respiratoire pour un patient, Toutefois, étant donné les dimensions réduites d'un tel appareil nasal qui doit être placé au moins en partie dans les narines, l'efficacité de la dérivation due à un tube intérieur creux sera moindre étant donné son diamètre plus réduit que dans les autres applications.

Un appareil nasal d'assistance respiratoire met en oeuvre un dispositif selon l'invention par narine, les deux dispositifs étant disposés en parallèles. Cet appareil nasal est relié à un circuit d'alimentation en gaz respirable(s) et, éventuellement, en liquide d'humidification. De préférence, l'appareil comporte deux dispositifs de l'invention au sein d'une pièce monolithique. Des raccords tubulaires latéraux de chacun des dispositifs sont respectivement raccordés à des conduits d'alimentation, alimentés en parallèle en gaz respirable de ventilation par un dispositif d'alimentation, lui-même relié par une conduite à une source d'un tel gaz.

Optionnellement, si l'on souhaite une mesure de pression, dans l'un des canaux principaux ou dans les deux canaux principaux, est introduit un tube capillaire dont l'extrémité distale est pourvue d'une échancrure latérale et dont la longueur est égale à plusieurs fois le diamètre dudit tube capillaire. Cette échancrure latérale du tube capillaire est disposée dans la partie distale du canal principal et sert de prise de pression à cet endroit. Le tube capillaire transmet la pression au dispositif d'alimentation et à un manomètre à eau.

Optionnellement, si l'on souhaite une humidification, chaque élément tubulaire est associé à un conduit d'apport sous forme d'un conduit latéral débouchant dans une partie intermédiaire conique du canal principal correspondant, entre une partie de plus grand diamètre et une partie de plus petit diamètre, distale, dudit canal principal. L'extrémité desdits conduits latéraux fait saillie à l'intérieur du canal principal et est taillée en biseau. L'inclinaison dudit biseau est opposée à celle de la partie intermédiaire conique. Les conduits latéraux sont reliés en parallèle à un réservoir contenant un liquide, par exemple de l'eau, éventuellement additionnée de médicaments ou analogues, et sont alimentés en liquide à partir de ce réservoir, par exemple par gravité et/ou capillarité.

On peut prévoir, lorsqu'après une expiration le patient commence à inspirer, que le dispositif d'alimentation reçoive la variation de pression correspondante par le tube capillaire et permettre d'adresser au patient des jets, selon les besoins, continuels ou impulsionnels, de gaz respirable(s) par l'intermédiaire des conduits d'alimentation. Les jets de gaz respirable(s), formés à l'intérieur de chaque canal principal, sont défléchis par les parties intermédiaires coniques en entraînant au passage les gouttelettes du liquide accrochées aux biseaux d'extrémités des conduits latéraux et s'humidifient. En revanche, lorsqu'après une inspiration, le patient commence à expirer, l'appareil d'alimentation est commandé à l'arrêt par la pression transmise par le tube capillaire et le patient peut librement expirer à travers les canaux principaux.

On comprend que ce moyen de contrôle agissant en fonction des phases respiratoires sur la fourniture de gaz respirable peut être mis en oeuvre dans d'autres applications que nasale du dispositif de l'invention.

Des rondelles de mousse peuvent être enfilées sur les extrémités distales des éléments tubulaires et servent alors de butée souple à l'enfoncement de la pièce monolithique dans les narines du patient.

La variante 205 du dispositif, détaillée sur les figures 7 à 10, comporte un canal principal interne 207 et, en partie médiane, une paroi conique 208, saillant à l'intérieur dudit canal principal 207. La paroi conique 208 a pour objet de défléchir, en direction de l'axe du canal principal 207, des jets de gaz respirable(s) injectés par des canaux auxiliaires 209, alimentés à partir d'un embout d'amenée 210, par l'intermédiaire d'une chambre annulaire périphérique 211.

Le dispositif 205 comporte un tube intérieur 50 au centre du canal principal 207, coaxial à l'axe 16 de ce dernier. Le tube intérieur, qui est creux, forme, d'une part, un brise jet du fait qu'il traverse la zone des jets défléchis produits par les orifices des canaux auxiliaires et, d'autre part, une dérivation grâce à sa lumière 54 étendue entre ses deux extrémités 52, 53.

Dans cette variante, les ailettes 51 sont étendues entre le tube intérieur 50 et un corps tubulaire 55 qui est inséré dans l'élément tubulaire 4 par l'extrémité proximale de ce dernier. Le tube intérieur 50, les ailettes 51 et le corps tubulaire 55 peuvent dans certaines variantes former une pièce monobloc introduite dans l'extrémité proximale de l'élément tubulaire 4. Le corps tubulaire 55 comporte ici des éléments structuraux destinés à former en combinaison avec d'autres éléments structuraux de l'élément tubulaire 4 les canaux auxiliaires 209 et leurs orifices de sortie.

Par ailleurs, du côté distal, le dispositif 205 comporte une chambre périphérique annulaire 212, coaxiale audit dispositif 205. La chambre périphérique annulaire 212 débouche côté distal par un passage annulaire distal 213. Dans le cas où le dispositif 205 est installé sur un masque facial, la chambre périphérique annulaire 212 communique avec l'intérieur du masque par ce passage annulaire distal 213. La chambre périphérique annulaire 212 est en relation avec, côté proximal, un embout de sortie 214.

Un filtre 215, fibreux ou poreux, par exemple en coton, mousse synthétique, ou autre, est disposé dans la chambre périphérique annulaire 212, pour amortir les turbulences gazeuses et, par suite, les trop fortes variations de pression. En effet, l'embout de sortie 214 peut être relié, par exemple, à un analyseur de gaz et/ou à un dispositif de mesure de pression. Bien entendu, les liaisons entre l'embout de sortie 214 et l'analyseur de gaz et/ou à le dispositif de mesure de pression sont prévues pour que le prélèvement de gaz à analyser par la liaison n'ait pas d'influence sur l'analyse et/ou la mesure de pression, via la liaison.

Ainsi, le praticien assistant le patient connaît en permanence la composition du gaz dans le masque, notamment sa teneur en gaz carbonique, et la pression à l'intérieur dudit masque. Il peut donc prendre les mesures d'intervention appropriées en fonction desdites composition et pression du gaz.

On comprend que cette variante 205 peut être réalisée de manière différente tout en assurant les mêmes fonctions d'assistance respiratoire et de prise pour mesures. De plus, le dispositif peut également comporter en plus, des moyens ou conduits d'injection de médicaments et/ou d'eau, notamment pour nébulisation.

Plus généralement le dispositif de l'invention peut trouver de nombreuses autres applications, par exemple en combinaison avec une sonde nasale, une sonde buccale, une sonde trachéale, etc.

On va maintenant détailler le positionnement du tube intérieur par rapport aux jets défléchis de gaz sortant des orifices de sortie des canaux auxiliaires. A cette fin, la Figure 11 détaille plus spécifiquement l'interaction entre le tube intérieur et les jets.

Sur la Figure 11, on a représenté seulement une partie d'un élément tubulaire 4, plus particulièrement une partie comprise sensiblement entre les orifices de sortie 17 des canaux auxiliaires 8 et l'extrémité distale 7 (vers la droite de la Figure mais non visible) dudit élément tubulaire. Le tube intérieur 50 est disposé centralement dans le canal principal 5 coaxialement à l'axe 16 de ce dernier.

Afin de simplifier les explications et sans que cela corresponde forcément à la réalité on a supposé que le jet défléchi subissait des réflexions spéculaires sur la surface externe du tube intérieur et la surface interne de l'élément tubulaire. On comprend que cette réflexion spéculaire des jets défléchis ne doit pas se produire en réalité du fait de la perte d'énergie des gaz par rencontre sur le tube intérieur et des turbulences qui sont créées dans le canal principal mais c'est une approximation simple pour la suite des explications. Les ailettes de maintien supportant du tube intérieur n'ont pas été représentées également pour des raisons de simplification mais on peut en prévoir côté proximal et/ou distal de l'élément tubulaire, ceci fonction de la longueur du tube intérieur.

On considère que c'est le long des jets que la pression des gaz est la plus forte, en particulier pour le jet défléchi initial 60.1. Après la première réflexion sur la surface externe du tube intérieur cela devient de moins en moins vrai du fait des turbulences qui tendent à homogénéiser les pressions. Toutefois, pour simplifier les explications on considère que le long des axes des jets on a une pression supérieure au moins dans une zone proche des orifices de sortie 17 des canaux auxiliaires 8. Après une première réflexion sur le tube intérieur 50 le jet est supposé renvoyé le long de l'axe 60.2 vers la surface interne de l'élément tubulaire ou il est réfléchi sur un axe de jet 60.3.

Le tube intérieur 50 est creux et est ouvert à ses deux extrémités proximale 53 et distale 52. Pour l'extrémité distale 52 du tube intérieur 50 on a représenté plusieurs possibilités de position d'extrémité : 52.1, 52.2 ou 52.3. Dans tous les cas on remarque que l'extrémité distale du tube intérieur se trouve à un niveau tel que l'axe du jet est éloigné et donc dans une zone où il n'y a pas une pression particulièrement élevée par rapport à la pression homogène que l'on peut rencontrer plus loin du côté de l'extrémité distale du canal principal. Ceci est en effet préférable pour éviter, en cas d'extrémité distale du tube intérieur dans une zone de pression élevée, que la lumière 54 forme en fait une fuite des gaz vers le côté proximal et l'environnement. En outre, il y a un risque de génération de bruit, notamment sifflement, si l'extrémité distale du tube intérieur se termine au niveau d'un axe de jet, en particulier le premier 60.1. On comprend bien que si l'extrémité distale du tube intérieur est installée, côté distal de l'élément tubulaire, bien à distance des orifices de sortie des canaux auxiliaires, dans une zone où la pression des gaz s'est uniformisée, les risques potentiels évoqués ci-dessus disparaissent. C'est ainsi que l'exsufflation/expiration se fait prioritairement par la lumière du tube intérieur. On peut interpréter le fonctionnement du dispositif de l'invention avec tube intérieur creux comme une redistribution des flux de gaz : les turbulences engendrées par la rencontre des jets entre eux des dispositifs classiques sont remplacées par une redistribution des flux : un « couloir » autour du tube intérieur permet l'insufflation vers le patient et un autre « couloir » dans la lumière du tube intérieur permet l'exsufflation du patient.

L'extrémité proximale 53 tu tube intérieur 50 a été représentée sensiblement au niveau des orifices de sortie 17 des canaux auxiliaires. Dans des variantes on peut la placer plus du côté de l'extrémité proximale de l'élément tubulaire. Cette dernière solution peut correspondre à celle des Figures 1 ou 7.

On va maintenant décrire un mode de réalisation particulier du dispositif qui dérive de celui décrit en relation avec les Figures 7 à 10, c'est-à-dire un dispositif comportant un corps tubulaire 55 inséré dans la partie proximale de l'élément tubulaire 4.

Sur la Figure 12, le corps tubulaire 55 est vu en perspective, l'extrémité proximale 6 étant vers l'observateur. Une pièce monobloc à tube intérieur 50 est encastrée dans le corps tubulaire 55. La pièce monobloc est constituée du tube intérieur 50 creux, des ailettes 51 et de l'anneau circulaire 56. Dans cet exemple seulement deux ailettes 51 sont réalisées. Le corps tubulaire 55 destiné à s'insérer dans l'extrémité proximale de l'élément tubulaire comporte des éléments structuraux destinés à former certaines parties des canaux auxiliaires 209, des orifices de sortie desdits canaux auxiliaires et de la chambre annulaire périphérique 211.

On a schématisé sur la Figure 13 les axes des jets défléchis initiaux 60.1 provenant de deux des orifices de sortie des canaux auxiliaires. Si le tube intérieur avait été omis, les axes se seraient rencontrés au point de rencontre R comme cela est le cas dans les dispositifs classiques du type Boussignac. Du fait de la présence du tube intérieur 50, les jets heurtent le tube intérieur, d'où l'effet brise jet dudit tube intérieur.

Sur ces Figures 12 et 13, l'anneau circulaire 56 s'applique par sa surface extérieure sur la surface interne de l'élément tubulaire 4 car la pièce monobloc s'encastre dans l'extrémité proximale du corps tubulaire 55 mais, dans des variantes, l'anneau circulaire 56 pourra s'appliquer par sa surface extérieure sur la surface interne du corps tubulaire 55.

Dans un dispositif de ce type et pour une CPAP de 13 mm, la section interne de passage du tube intérieur est de 29 mm², ce qui laisse autour du tube intérieur dans le canal principal une section de passage de 88 mm², d'où un rapport de surfaces transversales de passage d'environ 1/3. Toujours dans ce type de dispositif, une longueur de tube intérieur de 43,5 mm environ s'est avérée satisfaisante, l'extrémité distale du tube intérieur étant à environ 16 mm de la zone de rencontre des jets avec le tube intérieur, jets défléchis sortants directement des orifices de sortie des canaux auxiliaires. Les orifices de sortie des canaux auxiliaires sont ici en bandes semi-circulaires de 0,25 mm d'épaisseur sur une longueur d'arc d'environ 1 mm.

Dans d'autres variantes de réalisation, le dispositif peut mettre en oeuvre un seul jet latéral défléchi et dans ce cas le tube intérieur brise jet fera s'enrouler le jet autour de lui en spirale du fait de l'inclinaison du jet défléchi. La section de passage du tube intérieur peut être adaptée aux applications prévues pour le dispositif. A la limite, la section de passage peut être nulle, le tube intérieur étant alors seulement un brise jet sans dérivation.

De préférence, le tube intérieur, notamment lorsqu'il est sous la forme d'une pièce monobloc, comporte, tout comme les parties du dispositif auxquelles il est en contact, des moyens complémentaires de positionnement et/ou détrompeurs, voire de blocage/verrouillage afin que son insertion et maintient soient assurés de façon prédéfinie. Par exemple, on peut voir que sur les Figures 12 et 13 que la pièce monobloc s'encastre jusqu'à buter sur des nervures 58 du corps tubulaire et ne peut pas être poussée vers le côté distal au-delà desdites nervures.

Sur les Figures 12 et 13, les pattes 57 passent sous l'anneau circulaire 56. Ces pattes 57 peuvent présenter à leurs extrémités proximales des ergots empêchant le retrait de la pièce monobloc une fois celle-ci encastrée. On comprend que d'autres moyens équivalents sont utilisables.

L'élément tubulaire constitutif des modes de réalisation du dispositif selon l'invention peut être réalisé en toute matière déjà utilisée dans les sondes respiratoires, par exemple en un chlorure de polyvinyle, avec un éventuel revêtement de silicone ou en acier permettant les injections à pression élevée.

Le dispositif à tube intérieur peut être amovible ou être fixé à demeure/faire partie d'un dispositif de valve CPAP de type Boussignac. Dans le cas d'un dispositif amovible, il peut être relié à la CPAP par une languette souple. Préférentiellement, le dispositif est conçu de façon intégrée à une CPAP de type Boussignac, donc sans être une pièce rapportée.

Le tube intérieur creux a une épaisseur de paroi la plus faible possible afin de ne pas gêner l'expiration et de ne pas engendrer de surpression. Par exemple, une épaisseur de paroi du tube intérieur de 0,7 mm est prévue. Le tube intérieur est conçu avec un matériau approprié qui n'entraine pas de résonnance, par exemple en : silicone, POM, ABS.

De préférence, le tube intérieur doit se trouver proche des orifices de sortie des jets pour limiter le bruit de façon optimale sans toutefois boucher ces orifices ou gêner la sortie des jets pour maintenir une pression suffisante dans le dispositif. Par exemple, pour un diamètre externe de 7,0 mm du tube intérieur, la distance de la surface externe du tube intérieur à un orifice de sortie de jet, distance transversale dans le dispositif, est comprise entre 1 mm et 5 mm.

Comme on l'a vu, le tube intérieur doit avoir une longueur suffisante pour se retrouver à l'encontre des jets et il peut même être un peu plus long afin de guider les gaz le long de sa paroi. Par exemple, on prévoit une longueur de tube intérieur comprise entre 30 mm et 55 mm, mais la longueur du tube intérieur peut être réduite à 5 mm ou 10 mm par exemple si il est intégré dans une CPAP de type Boussignac.

Bien entendu, les dimensions du dispositif selon l'invention peuvent être très variables, essentiellement en fonction de l'application, de la voie de mise en place de l'élément tubulaire et de la taille du patient, qui peut être un adulte, un enfant, un nourrisson ou un prématuré.

On a pu montrer qu'avec le dispositif de l'invention à tube intérieur on ne notait pas de modification significative des performances de la valve CPAP de type Boussignac par rapport à une valve CPAP de type Boussignac classique, c'est-à-dire sans tube intérieur, sur les paramètres suivants : pression générée, résistance à l'expiration, taux de FiO2. Par contre on a pu obtenir une diminution significative de la pression acoustique.

Le dispositif de l'invention peut être utilisé dans les tentatives de réanimation de personnes en arrêt respiratoire et/ou cardiaque. Ces personnes peuvent produire des rejets liquides de type expectorations, hémorragies, hémoptysies, voire vomissements lorsqu'elles ne sont pas encore intubées, etc. qui peuvent passer dans les voies respiratoires et être expulsés à travers le dispositif. Grâce à l'invention, on réduit le risque de les disperser par la sortie/extrémité proximale et le risque de provoquer des souillures possiblement contaminantes. De plus, grâce au tube intérieur on peut obtenir une réduction du bruit généré par rapport aux dispositifs classiques sans tube intérieur.

Dans certaines conditions, on peut prévoir en outre des moyens de filtrage/retenue de ces rejets liquides côté extrémité proximale du dispositif. Ces moyens peuvent être un filtre, par exemple un adaptateur dans lequel une masse de filtrage est installée ou peut l'être, ladite masse pouvant par exemple être une compresse dont la texture permet le passage des gaz. Ces moyens peuvent être un décanteur avec un récipient de décantation, de préférence à distance du dispositif et relié à ce dernier par un tube souple. Dans le cas où le dispositif comporte en outre des moyens fonctionnels additionnels côté proximal, par exemple des moyens de freinage, alors, de préférence, les moyens de filtrage/retenue sont disposés entre le dispositif et ces moyens fonctionnels additionnels. Dans une variante plus simple, un tube est simplement relié à l'extrémité proximale du dispositif par sa première extrémité et l'autre extrémité du tube est positionnée à un emplacement où d'éventuelles souillures sont sans importance et/ou peuvent être récoltées.

Enfin, dans une variante de mise en oeuvre présentée dans la demande de brevet WO2008113913 et qui peut être employée dans le cadre de la présente invention, le dispositif comporte en outre des moyens de freinage spontané de l'entrée d'air extérieur dans le canal principal via son extrémité proximale. Ces moyens peuvent être installés à demeure sur le dispositif et faire partie intégrante de celui-ci ou, alors, être amovibles par raccordement sur l'extrémité proximale dudit dispositif. Grâce à ces moyens de freinage, il est possible d'utiliser le dispositif pour la réanimation d'une personne en état d'arrêt cardiaque. Dans ce cas, on utilise le dispositif à moyens de freinage pour la ventilation, par exemple au masque ou par intubation, et on exerce des compressions et des décompressions alternées sur la cage thoracique de ladite personne. Il se produit alors un freinage de l'entrée de l'air extérieur dans le dispositif et donc vers le patient, au début de chaque décompression. Il en résulte une baisse de pression pulmonaire dans la cage thoracique et donc sur le coeur et donc une aide à l'expansion cardiaque et au retour sanguin vers le coeur.

## Revendications

1. Dispositif d'assistance respiratoire (1) pour un patient comportant un élément tubulaire formant un canal principal (5) (207) qui est destiné à être relié par son extrémité distale (7) à une voie respiratoire du patient, ledit canal principal reliant à l'extérieur le système respiratoire dudit patient par son extrémité proximale (6), ledit dispositif comportant en outre au moins un canal auxiliaire (8) (209) permettant l'injection par un/des orifices de sortie distaux (17) du/des canaux auxiliaires de jet(s) de gaz respirable(s) destiné(s) à la ventilation dudit patient, le/lesdits orifices de sortie débouchant dans ledit canal principal au voisinage de l'extrémité distale de ce dernier, des moyens de déflexion (14b) (14a) permettant la déflexion des jets de gaz respirable(s) vers l'intérieur dudit canal principal et vers l'extrémité distale selon une inclinaison déterminée,
**caractérisé en ce qu'**il comporte en outre un tube intérieur coaxial centré dans le canal principal, ledit tube intérieur de longueur déterminée ayant une extrémité distale orientée vers l'extrémité distale de l'élément tubulaire et une extrémité proximale orientée vers l'extrémité proximale de l'élément tubulaire, ladite longueur déterminée étant telle que ledit tube intérieur est étendu au maximum entre les deux extrémités distale et proximale dudit dispositif d'assistance respiratoire, ledit tube intérieur coupant le/les axes des jets défléchis afin de former un brise jet vers le centre du canal principal dans une zone de rencontre desdits axes de jets défléchis et dudit tube intérieur, et
**en ce que** le tube intérieur est creux et est ouvert à ses deux extrémités dans le canal principal afin de former une dérivation entre l'extrémité distale et l'extrémité proximale du canal principal pour les gaz circulants dans ledit canal principal et notamment pour les gaz expirés par le patient.

2. Dispositif selon la revendication 1, **caractérisé en ce que** le tube intérieur est maintenu en position coaxiale à l'intérieur du canal principal par des ailettes étendues radialement entre la surface externe du tube intérieur et la surface interne de l'élément tubulaire, lesdites ailettes étant absentes dans la zone des jets défléchis.

3. Dispositif selon la revendication 2, **caractérisé en ce que** les ailettes et le tube intérieur forment une pièce monobloc insérée dans l'élément tubulaire (4) ou dans un corps tubulaire (55) lui-même inséré dans l'élément tubulaire (4).

4. Dispositif selon la revendication 3, **caractérisé en ce que** la pièce monobloc à ailettes et tube intérieur est introduite dans l'élément tubulaire par l'extrémité proximale de ce dernier, les ailettes étant disposées seulement du côté de l'extrémité proximale dudit élément tubulaire et formant en outre un obstacle à un bouchage du canal principal.

5. Dispositif selon la revendication 3 ou 4, **caractérisé en ce que** la pièce monobloc à ailettes et tube intérieur comporte à la périphérie radiale des ailettes un anneau circulaire dont la surface extérieure est au contact de la surface interne de l'élément tubulaire (4) ou d'un corps tubulaire (55) inséré dans l'élément tubulaire (4).

6. Dispositif selon la revendication 5, **caractérisé en ce que** l'élément tubulaire comporte des éléments structuraux destinés à former à eux seuls ou en combinaison avec d'autres éléments structuraux de l'anneau circulaire ou du corps tubulaire, en partie ou totalité, au moins le/les canaux auxiliaires.

7. Dispositif selon la revendication 5, **caractérisé en ce que** l'anneau circulaire ou le corps tubulaire comporte des éléments structuraux destinés à former à eux seuls ou en combinaison avec d'autres éléments structuraux de l'élément tubulaire, en partie ou totalité, le/les canaux auxiliaires et une partie du/des orifices de sortie des canaux auxiliaires.

8. Dispositif selon l'une quelconque des revendications précédentes, **caractérisé en ce que** le/les orifices de sortie distaux du/des canaux auxiliaires sont ponctuels et/ou sont en bande(s) annulaire ou semi-annulaires, et **en ce que** le diamètre des orifices ponctuels ou l'épaisseur des orifices en bande(s) est inférieure à 150 microns.

9. Dispositif selon l'une quelconque des revendications précédentes, **caractérisé en ce que** l'extrémité distale du tube intérieur se termine dans le canal principal approximativement au niveau de l'extrémité distale dudit dispositif d'assistance respiratoire et que l'extrémité proximale du tube intérieur se termine dans le canal principal approximativement au niveau de l'extrémité proximale dudit dispositif d'assistance respiratoire.

10. Dispositif selon l'une quelconque des revendications précédentes, **caractérisé en ce qu'**il est à double flux inversés permettant de favoriser l'expiration aussi bien que l'inspiration d'un patient, le dispositif comportant au moins un canal auxiliaire supplémentaire (8.2), indépendant du/des premiers canaux auxiliaires (8.1) des jets de l'extrémité distale du canal principal, et relié à une source de gaz sous pression, ledit au moins un canal auxiliaire supplémentaire débouchant dans le canal principal au voisinage de l'extrémité proximale de ce dernier, et au moins l'/les extrémités proximales dudit/desdits canaux auxiliaires supplémentaires débouchant par des orifices de sortie proximaux respectifs dans le canal principal est/sont parallèle à celui-ci, tandis que, en regard de chaque orifice de sortie proximal du canal auxiliaire supplémentaire correspondant, sont prévus des moyens pour la déflexion du jet de gaz traversant ce dernier en direction de l'intérieur dudit canal principal afin de former des jets d'extrémité proximale du canal principal, et
**en ce que** les deux zones de jets défléchis correspondant respectivement aux jets de l'extrémité distale du canal principal et aux jets de l'extrémité proximale du canal principal comportent chacune un tube intérieur brise jet et de dérivation, ledit tube intérieur pouvant être étendu pour être unique et commun aux deux zones ou chacune des zones comportant son tube intérieur propre.

11. Dispositif selon l'une quelconque des revendications précédentes, **caractérisé en ce qu'**il comporte en outre des moyens de freinage d'entrée de gaz d'inspiration de l'extérieur vers le patient et **en ce que** les moyens de freinage sont configurés pour ne freiner le passage des gaz que pendant une phase d'inspiration qui est le début de l'inspiration afin de provoquer une dépression intrathoracique favorable au retour veineux vers le coeur.

12. Dispositif selon la revendication 2, **caractérisé en ce qu'**il comporte intérieurement un indicateur visuel de direction et force du flux gazeux passant par le dispositif, ledit indicateur étant une bandelette souple en matière plastique fixée à une de ses deux extrémités à une ailette de maintien du tube intérieur.

13. Appareil nasal d'assistance respiratoire (100) pour un patient, **caractérisé en ce qu'**il comporte, pour chaque narine un dispositif selon l'une quelconque des revendications précédentes, les deux dispositifs étant alimentés en gaz respirable(s) en parallèle, chaque dispositif comportant un élément tubulaire formant un canal principal qui est destiné à être relié par son extrémité distale au système respiratoire du patient par une de ses narines, ledit canal principal reliant à l'extérieur le système respiratoire dudit patient par son extrémité proximale, ledit dispositif comportant en outre au moins un canal auxiliaire permettant l'injection de jets de gaz respirable(s) destiné(s) à la ventilation dudit patient par des orifices de sortie distaux du/des canaux auxiliaires, lesdits orifices de sortie débouchant dans ledit canal principal au voisinage de l'extrémité distale de ce dernier, des moyens de déflexion permettant la déflexion des jets de gaz vers l'intérieur dudit canal principal, et **en ce qu'**il comporte pour chacun des dispositifs un tube intérieur brise jet et de dérivation.

14. Masque d'assistance respiratoire (23) (201), **caractérisé en ce qu'**il est muni d'un dispositif selon l'une quelconque des revendications 1 à 12.

## Patentansprüche

1. Atemhilfevorrichtung (1) für einen Patienten, die ein einen Hauptkanal (5) (207) bildendes rohrförmiges Element aufweist, das dazu bestimmt ist, mit seinem distalen Ende (7) an einen Atemweg des Patienten angeschlossen zu werden, wobei der Hauptkanal durch sein proximales Ende (6) das Atemsystem des Patienten mit der Umgebung verbindet, wobei die Vorrichtung außerdem wenigstens einen Hilfskanal (8) (209) aufweist, der das Einspritzen eines Strahls (oder von Strahlen) eines (oder von) für die Beatmung des Patienten bestimmten Atemgases (Atemgasen) durch ein distales Ausgangsloch (oder distale Ausgangslöcher) (17) des Hilfskanals (oder der Hilfskanäle) ermöglicht, wobei das Ausgangsloch (die Ausgangslöcher) im Bereich des distalen Endes des Hauptkanals in letzteren mündet (münden), wobei Umlenkmittel (14b) (14a) das Umlenken der Atemgasstrahlen zum Inneren des Hauptkanals und zum distalen Ende mit einer bestimmten Neigung ermöglichen,
**dadurch gekennzeichnet, daß** sie außerdem ein im Hauptkanal zentriertes koaxiales inneres Rohr aufweist, wobei das innere Rohr von bestimmter Länge ein zum distalen Ende des rohrförmigen Elements gerichtetes distales Ende und ein zum proximalen Ende des rohrförmigen Elements gerichtetes proximales Ende aufweist, wobei die bestimmte Länge derart ist, daß sich das innere Rohr maximal zwischen dem distalen und dem proximalen Ende der Atemhilfevorrichtung erstreckt, wobei das innere Rohr die Achse(n) der abgelenkten Strahlen schneidet, um einen Strahlenbrecher zur Mitte des Hauptkanals in einem Auftreffbereich der abgelenkten Strahlachsen und des inneren Rohrs zu bilden, und
dadurch, daß das innere Rohr hohl ist und an seinen beiden Enden im Hauptkanal offen ist, um für die im Hauptkanal fließenden Gase und insbesondere für die vom Patienten ausgeatmeten Gase eine Ableitung zwischen dem distalen Ende und dem proximalen Ende des Hauptkanals zu bilden.

2. Vorrichtung gemäß Anspruch 1, **dadurch gekennzeichnet, daß** das innere Rohr im Inneren des Hauptkanals durch sich zwischen der äußeren Oberfläche des inneren Rohrs und der inneren Oberfläche des rohrförmigen Elements radial erstreckende Flügel in koaxialer Position gehalten wird, wobei die Flügel im Bereich der abgelenkten Strahlen nicht vorhanden sind.

3. Vorrichtung gemäß Anspruch 2, **dadurch gekennzeichnet, daß** die Flügel und das innere Rohr ein in das rohrförmige Element (4) oder in einen rohrförmigen Körper (55), der selbst in das rohrförmige Element (4) eingesetzt ist, eingesetztes einstückiges Teil bilden.

4. Vorrichtung gemäß Anspruch 3, **dadurch gekennzeichnet, daß** das einstückige Teil aus Flügeln und innerem Rohr von der Seite des proximalen Endes des rohrförmigen Elements her in letzteres eingeführt ist, wobei die Flügel nur auf der Seite des proximalen Endes des rohrförmigen Elements angeordnet sind und ein Hindernis gegen eine Verstopfung des Hauptkanals bilden.

5. Vorrichtung gemäß Anspruch 3 oder 4, **dadurch gekennzeichnet, daß** das einstückige Teil aus Flügeln und innerem Rohr am radialen Umfang der Flügel einen kreisförmigen Ring aufweist, dessen äußere Oberfläche mit der inneren Oberfläche des rohrförmigen Elements (4) oder eines in das rohrförmige Element eingeführten rohrförmigen Körpers (55) in Kontakt ist.

6. Vorrichtung gemäß Anspruch 5, **dadurch gekennzeichnet, daß** das rohrförmige Element Strukturelemente aufweist, die dazu bestimmt sind, für sich allein genommen oder in Kombination mit anderen Strukturelementen des kreisförmigen Rings oder des rohrförmigen Körpers, teilweise oder ganz, wenigstens den Hilfskanal (die Hilfskanäle) zu bilden.

7. Vorrichtung gemäß Anspruch 5, **dadurch gekennzeichnet, daß** der kreisförmige Ring oder der rohrförmige Körper Strukturelemente aufweist, die dazu bestimmt sind, für sich allein genommen oder in Kombination mit anderen Strukturelementen des rohrförmigen Elements, teilweise oder ganz, wenigstens den Hilfskanal (die Hilfskanäle) und einen Teil des/der Ausgangslöcher der Hilfskanäle zu bilden.

8. Vorrichtung gemäß einem der vorangehenden Ansprüche, **dadurch gekennzeichnet, daß** das/die distalen Ausgangslöcher des/der Hilfskanäle punktförmig und/oder in ringförmigem oder halbringförmigem Band (ringförmigen oder halbringförmigen Bändern) und daß der Durchmesser der punktförmigen Löcher oder die Dicke der Löcher als Band (oder Bänder) kleiner als 150 µm ist.

9. Vorrichtung gemäß einem der vorangehenden Ansprüche, **dadurch gekennzeichnet, daß** das distale Ende des inneren Rohrs im Hauptkanal ungefähr im Bereich des distalen Endes der Atemhilfevorrichtung endet und daß das proximale Ende des inneren Rohrs im Hauptkanal ungefähr im Bereich des proximalen Endes der Atemhilfevorrichtung endet.

10. Vorrichtung gemäß einem der vorangehenden Ansprüche, **dadurch gekennzeichnet, daß** sie vom Typ zweier gegenläufiger Ströme ist, was sowohl das Ausatmen als auch das Einatmen eines Patienten fördert, wobei die Vorrichtung wenigstens einen zusätzlichen Hilfskanal (8.2) aufweist, der von dem/den ersten Hilfskanälen (8.1) der Strahlen des distalen Endes des Hauptkanals unabhängig ist und mit einer Quelle unter Druck stehenden Gases verbunden ist, wobei der wenigstens eine zusätzliche Hilfskanal in der Nähe des proximalen Endes des Hauptkanals in letzteren mündet, und wobei wenigstens das/die proximalen Enden des/der über jeweilige proximale Ausgangslöcher in den Hauptkanal einmündenden zusätzlichen Hilfskanäle zu diesem parallel ist/sind, während gegenüber jedem proximalen Ausgangsloch des entsprechenden zusätzlichen Hilfskanals Mittel zum Umlenken des letzteren in Richtung zum Inneren des Hauptkanals durchfließenden Gasstrahls vorgesehen sind, um Strahlen des proximalen Endes des Hauptkanals zu bilden, und dadurch, daß die beiden Bereiche abgelenkter Strahlen, die den Strahlen des distalen Endes des Hauptkanals bzw. den Strahlen des proximalen Endes des Hauptkanals entsprechen, jeweils ein inneres Rohr zum Brechen und Ablenken der Strahlen aufweisen, wobei sich das innere Rohr so erstrecken kann, daß es das einzige und für beide Bereiche gemeinsame Rohr ist oder daß jeder Bereich sein eigenes inneres Rohr aufweist.

11. Vorrichtung gemäß einem der vorangehenden Ansprüche, **dadurch gekennzeichnet, daß** sie außerdem Mittel zum Bremsen des Einströmens des Einatmungsgases von außen zum Patienten hin aufweist und daß die Bremsmittel so ausgelegt sind, daß sie das Durchströmen der Gase nur während einer Einatmungsphase bremsen, die der Anfang des Einatmens ist, um im Brustkorb einen Unterdruck zu erzeugen, der den Rückfluß der Venen zum Herzen hin fördert.

12. Vorrichtung gemäß Anspruch 2, **dadurch gekennzeichnet, daß** sie im Inneren eine Sichtanzeige für die Richtung und die Stärke des durch die Vorrichtung fließenden Gasflusses aufweist, wobei die Anzeige ein flexibles Bändchen aus einem Plastikmaterial ist, das mit einem seiner Enden an einem Flügel zum Stützen des Inneren Rohrs befestigt ist.

13. Nasales Atemhilfegerät (100) für einen Patienten, **dadurch gekennzeichnet, daß** es für jedes Nasenloch eine Vorrichtung gemäß einem der vorangehenden Ansprüche aufweist, wobei die beiden Vorrichtungen mit Atemgas(en) parallel versorgt werden, wobei jede Vorrichtung ein einen Hauptkanal bildendes rohrförmiges Element aufweist, das dazu bestimmt ist, mit seinem distalen Ende über eins der Nasenlöcher des Patienten an dessen Atemsystem angeschlossen zu werden, wobei der Hauptkanal durch sein proximales Ende das Atemsystem des Patienten mit der Umgebung verbindet, wobei die Vorrichtung außerdem wenigstens einen Hilfskanal aufweist, der das Einspritzen eines Strahls (oder von Strahlen) eines (oder von) für die Beatmung des Patienten bestimmten Atemgases (Atemgasen) durch ein distales Ausgangsloch (oder distale Ausgangslöcher) des Hilfskanals (oder der Hilfskanäle) ermöglicht, wobei die Ausgangslöcher) im Bereich des distalen Endes des Hauptkanals in letzteren münden, wobei Umlenkmittel das Umlenken der Gasstrahlen zum Inneren des Hauptkanals ermöglichen, und dadurch, daß jede der Vorrichtungen ein inneres Rohr zum Brechen und Ablenken der Strahlen aufweist.

14. Atemhilfemaske (23) (201), **dadurch gekennzeichnet, daß** sie mit einer Vorrichtung gemäß einem der Ansprüche 1 bis 12 versehen ist.

## Claims

1. A respiratory assistance device (1) for a patient, including a tubular element forming a main channel (5) (207) that is intended to be connected by its distal end (7) to an airway of the patient, said main channel connecting the respiratory system of said patient to the outside by its proximal end (6), said device further including at least one auxiliary channel (8) (209) allowing the injection through one/several distal outlet orifice(s) (17) of the auxiliary channel(s) of jet(s) of breathable gas(es) intended for the ventilation of said patient, said outlet orifice(s) emerging into said main channel in the vicinity of the distal end of the latter, deflection means (14b) (14a) allowing the deflection of the jets of breathable gas(es) towards the inside of said main channel and towards the distal end according to a determined inclination,
**characterized in that** it further includes a coaxial inner tube centred in the main channel, said inner tube of determined length having a distal end turned towards the distal end of the tubular element and a proximal end turned towards the proximal end of the tubular end, said determined length being such that said inner tube is extended at the maximum between the two distal and proximal ends of said respiratory assistance device, said inner tube crossing the axis(es) of the deflected jets in order to form a jet breaker towards the centre of the main channel in a zone of meeting of said deflected jet axes and of said inner tube, and
**in that** the inner tube is hollow and open at the two ends thereof in the main channel in order to form a by-pass between the distal end and the proximal end of the main channel for the gases circulating in said main channel and in particular for the gases breathed out by the patient.

2. The device according to claim 1, **characterized in that** the inner tube is held in coaxial position inside the main channel by fins extending radially between the external surface of the inner tube and the internal surface of the tubular element, said fins being absent in the zone of the deflected jets.

3. The device according to claim 2, **characterized in that** the fins and the inner tube form a single-piece part inserted into the tubular element (4) or into a tubular body (55) itself inserted into the tubular element (4).

4. The device according to claim 3, **characterized in that** the single-piece part with fins and inner tube is introduced into the tubular element through the proximal end of the latter, the fins being arranged only on the side of the proximal end of said tubular element and moreover forming an obstacle to a clogging of the main channel.

5. The device according to claim 3 or 4, **characterized in that** the single-piece part with fins and inner tube includes, at the radial periphery of the fins, a circular ring whose external surface is in contact with the internal surface of the tubular element (4) or a tubular body (55) inserted into the tubular element (4).

6. The device according to claim 5, **characterized in that** the tubular element includes structural elements intended to form alone or in combination with other structural elements of the circular ring or of the tubular body, in part or in totality, at least the auxiliary channel(s).

7. The device according to claim 5, **characterized in that** the circular ring or the tubular body includes structural elements intended to form alone or in combination with other structural elements of the tubular element, in part or in totality, the auxiliary channel(s) and a part of the outlet orifice(s) of the auxiliary channels.

8. The device according to any one of the preceding claims, **characterized in that** the distal outlet orifice(s) of the auxiliary channel(s) are punctual and/or are annular or semi-annular band-shaped, and **in that** the diameter of the punctual orifices or the thickness of the band-shaped orifices is lower than 150 micrometres.

9. The device according to any one of the preceding claims, **characterized in that** the distal end of the inner tube ends up in the main channel approximately at the distal end of said respiratory assistance device and **in that** the proximal end of the inner tube ends up in the main channel approximately at the proximal end of said respiratory assistance device.

10. The device according to any one of the preceding claims, **characterized in that** it is of the dual opposite flow type, making it possible to favour the breathing out as well as the breathing in of a patient, the device including at least one additional auxiliary channel (8.2), independent of the first auxiliary channel(s) (8.1) of the jets of the main channel distal end, and connected to a source of pressurized gas, said at least one additional auxiliary channel emerging into the main channel in the vicinity of the proximal end of the latter, and at least the proximal end(s) of said additional auxiliary channels emerging through respective proximal outlet orifices into the main channel is/are parallel to the latter, whereas, opposite each proximal outlet orifice of the corresponding additional auxiliary channel, are provided means for the deflection of the gas jet passing through the latter towards the inside of said main channel, in order to form jets of proximal end of the main channel, and
**in that** the two zones of deflected jets corresponding respectively to the jets of the main channel distal end and to the jets of the main channel proximal end each include a jet-breaking and by-passing inner tube, wherein said inner tube can be extended so as to be unique and common to the two zones or each of the zones includes its own inner tube.

11. The device according to any one of the preceding claims, **characterized in that** it further includes means for braking the entry of breathing-in gases from the outside to the patient and **in that** the braking means are configured so as to brake the passage of the gases only during a breathing-in phase that is the beginning of the breathing in, in order to cause an intrathoracic depression favourable to the venous return towards the heart.

12. The device according to claim 2, **characterized in that** it includes internally a visual indicator of direction and strength of the gaseous flow passing through the device, said indicator being a flexible strip made of plastic material, fastened at one of its two ends to a holding fin of the inner tube.

13. A nasal respiratory assistance apparatus (100) for a patient, **characterized in that** it includes, for each nostril, a device according to any one of the preceding claims, the two devices being fed with breathable gas(es) in parallel, each device including a tubular element forming a main channel that is intended to be connected by its distal end to the respiratory system of the patient through one of his nostrils, said main channel connecting the respiratory system of said patient to the outside by its proximal end, said device further including at least one auxiliary channel allowing the injection of jets of breathable gas(es) intended for the ventilation of said patient through distal outlet orifice(s) of the auxiliary channel(s), said outlet orifice(s) emerging into said main channel in the vicinity of the distal end of the latter, deflection means allowing the deflection of the jets of gas towards the inside of said main channel, and **in that** it includes for each of the devices a jet-breaking and by-passing inner tube.

14. A respiratory assistance mask (23) (201), **characterized in that** it is provided with a device according to any one of claims 1 to 12.
